# EUROPEAN PATENT APPLICATION

(11) **EP 4 443 163 A1**
(43) Date of publication of application: **09.10.2024**
(21) Application number: 23167220.5
(22) Date of filing: 07.04.2023
(51) Int. Cl.: G01N 33/68, G01N 33/50, G01N 33/574

(54) **METHOD FOR HIGH-THROUGHPUT QUALITATIVE AND QUANTITATIVE INTRACELLULAR PEPTIDOMICS OF MAMMALIAN CELLS**

(71) Applicant: Uniwersytet Gdanski, 80-309 Gdansk (PL)
(72) Inventor: Kote, Sachin, 80-116 Gdansk (PL); Marek-Trzonkowska, Natalia, 80-752 Gdansk (PL); Pirog, Artur, 80-312 Gdansk (PL); Faktor, Jakub, 80-170 Gdansk (PL)
(74) Representative: Czarnik, Maciej

(57) **Abstract**

The invention refers to an intracellular peptidomics sample preparation, qualitative and quantitative method for intracellular peptidome profiling in the *in vitro* cellular model based on amino acid sequencing with tandem mass spectrometry. The invention is simple, cost-effective, fast, and comprehensive for cellular peptidomics profiling. Moreover, the methods enable the discovery of intracellular peptides that can be used for diagnosis, biomarker discovery, screening of the peptides as drug targets, and predicting treatment response. Hence inventions provide valuable insights for understating the basic and applied biological questions.

## Description

The invention refers to an intracellular peptidomics sample preparation, qualitative and quantitative method for intracellular peptidome profiling in the *in vitro* cellular model based on amino acid sequencing with tandem mass spectrometry. The invention is simple, cost-effective, fast, and comprehensive for cellular peptidomics profiling. Moreover, the methods enable the discovery of intracellular peptides that can be used for diagnosis, biomarker discovery, screening of the peptides as drug targets, and predicting treatment response. Hence inventions provide valuable insights for understating the basic and applied biological questions.

The concept of peptidomics has been known for at least 20-30 years. It was first applied to specific classes of bioactive peptides, such as neuropeptides. Later, with the development of analytical technology, peptides were analyzed in body fluids and various secretions. The peptidome [1] is considered as low molecular weight peptides/proteins (less than 15 kilodaltons (kDa). Endogenous peptides primarily act as a messenger (cytokines, hormones, growth factors, etc.) and indicate various biological processes. Hence, these peptides can reflect the health or disease state of the person. In addition, the endogenous peptides provide insight into proteolytic activity, degradation, and degeneration, allowing the study of disease microenvironments.

Over the past decades, advanced technology allowed comprehensive peptidomics analyses. Particularly, label-free liquid chromatography with tandem mass spectrometry (LC-MS/MS) routinely identifies and quantifies thousands of peptides in a single run, providing an unprecedented opportunity to examine changes in the intracellular peptidome profile of a biological fraction or organism. Furthermore, downstream multi-bioinformatics reveal the functional understanding. Two causes primarily drove this particular direction - firstly, the expectation of bioactive, important peptides in reasonable concentrations, and secondly, limitations of the technology available at a certain moment. Intracellular peptidomics differs from proteomics, and the requirements for successfully identifying peptides are much higher with the peptidomics approach. Expected further applications mainly concern the discovery of novel bioactive peptides, which may arise from unexpected sources, such as protein degradation or translation of non-canonical open reading frames (ORFs). Studying protein degradation itself may generate very impactful biological information. Moreover, it is necessary to perform quantitative characterization of the whole intracellular peptidome to achieve this. This is a very complex mixture of peptides due to different lengths, with a high probability dominated by intermediates of protein degradation. Both of these traits must be addressed by the analytical method intended for its analysis. Firstly, a high dynamic range needed to be achieved to detect and quantify peptides in a mixture containing substantial amounts of, e.g., degradation products of abundant cytoskeletal proteins. Secondly, the applied LC/MS (Liquid Chromatography with tandem mass spectrometry) method must handle peptides of different mass and hydrophobicity, providing good chromatographic separation and further MS data acquisition as possible. Currently, applied methods rely primarily on differential solubility in organic solvents or partial protein denaturation [2], [3] by heat treatment. They often suffer from the poor recovery of intracellular peptides. Lack of hydrophobic interaction breaking agent on the denaturation step may hinder the possibility of recovering peptides tightly bound as ligands to other proteins. Here inventors show a way to largely overcome this problem, providing sample preparation guidelines enabling peptidomics analysis of readily available amounts of material.

Several biological questions still need to be comprehensively addressed in basic and applied sciences. Examples are the biological significance of protein degradation, degradation intermediates, and non-canonical translation products. Intracellular peptidome analysis is an underdeveloped field, mainly due to problems extracting low molecular weight peptides from the cell. Potential applications of cellular peptidomics arise directly from the novelty of the field. The most important aspect is that it enables the discovery of a novel repertoire of potentially interesting and essential molecules, yet similar to proteins. It allows the detection of novel drug targets, among them easily-druggable and specific ones. Intracellular peptides may also serve a biomarker role in diagnosis, disease prognosis, or assessment of the efficiency of the treatment. Besides the value in discovery-oriented applications, it can be a tool when protein degradation is a concern. Monitoring polypeptide's expression and subsequent degradation may be beneficial in several cases. In developing mRNA-based vaccines, it would be highly advantageous to know how the polypeptide product is expressed, how it is degraded, and what pathway leads to presentation of immunogenic peptides (antigenic peptides or immunopeptidome) on cell surfaces. A more common application might be monitoring of protein production. Analysis of peptides from the degradation of overexpressed protein may suggest steps to improve the yield and purity of the [4] final product. Furthermore, in the context of immunotherapy, it is necessary to understand the exact mechanism of antigenic peptide processing and sources of antigenic peptides. Presentation of major histocompatibility complex (MHC) ligands on the cell surface is the essential way of communicating the state of the cell to the immune system. Any disturbances in this process may be a potential cause of disease. For example, autoimmune disorders may emerge when the immune system incorrectly recognizes cells as non-self and attacks them. On the other hand, cancer cells may hide from immunity when no signal on the cell surface can provide an impulse for the immune system to kill the altered cells. Therefore, developing a method for detecting non-canonical peptide precursors presented by histocompatibility antigens (MHC) is highly demanded. Moreover, determining the peptide presentation pathway from the translation product to the MHC ligand in various disease scenarios will be necessary for developing vaccines, autoimmune disease therapies, cellular therapies, anticancer and antiviral therapies.

Intracellular peptide analysis is still a developing field, and inventors identify several areas that will significantly profit from the development.
1. The nature of intracellular peptidome is currently not well defined. There is no reliable information on the overall abundance of peptides of different lengths inside cells. Experiments done with differing extraction methods can result in dramatically different yields. To our knowledge, no experiments were performed to address this question precisely. In addition, peptidome is complex and characterized by a high concentration dynamic range, similar to proteome. This will pose another analytical challenge, akin to proteomic analysis, often requiring fractionation or targeted analysis to identify and quantify (poly) peptides of interest.
2. As mentioned, there is no consensus on the expected yield of intracellular peptide isolation. This suggests that considerable sample losses are affecting reported methods (Table 1). In the inventor opinion, there is a strong need to disrupt all noncovalent interactions and efficiently dissolve proteins and peptides prior to any attempt of size fractionation. The widespread belief is that cell lysis while using only one type of denaturing agent [5], [6] cannot provide optimal detection of peptides. However, the inventors have considered using only one type, i.e., urea as a denaturing agent, which also provides comprehensive intracellular peptides detection.
3. Previously described methods (Table 1) [7], [8], [9], [10], [5], [11], [12], [13], [6] for intracellular peptidomics analysis were characterized by low numbers of identified peptides and few quantitative methods for peptidome analysis were introduced. There are several reasons why this approach is restricted for comprehensive analysis, and these are as follows; a) enormous complexity because cells contain elements of all proteins produced in the living cells, b) high abundant proteins mask the detection of other proteins, especially low abundance proteins, and peptides, c) simultaneously limit of detection (LOD) of low abundant peptides with diagnostic potential, d) complicated and multistep sample preparation, instability of low abundant proteins or peptides (hormones or small secreted peptides), f) precipitation, heat treatment methods for the removal of impurities or high abundant proteins are overwhelming that compromise the number, yield of diagnostic cellular peptides identified and crucial biological information can be lost in the background noise.
4. Intracellular peptides are nonspecific, often modified, and exhibit a broad length range. There is no defined border between peptide and small protein. All these traits cause substantial problems in analysis. Firstly, separation and MS (tandem mass spectrometry) analysis conditions must fit longer and more hydrophobic peptides. In addition, longer peptides can bear more modified residues. The resulting mass spectra are complex and hard to identify.
5. As most of the available curated knowledge databases are gene- or protein-centric. This precludes efficient application of them in the analysis of peptidome. While source genes or proteins can be easily identified, especially for long, hence more specific peptides, there is no reason for them to be valid for a much shorter protein part. Therefore, additional bioinformatic analyses are highly demanded for high-quality data interpretation.

Inventors compared scientific literature with existing peptidomics and commonly used methods like proteomics, etc. In the known methods, only few reports [7], [8], [9], [10], [5], [11], [12], [13], [6] performed qualitative or quantitative intracellular peptidome analyses (Table 1). Hence, inventors consider peptidomes extracted from cultured cells, tissues, or whole small animals (Table 1). Inventors observe a variety of attempts to perform a size fractionation of polypeptides, usually by initial removal of most of the proteins by organic solvent or heat precipitation, followed by the ultracentrifugation step. Interestingly, none of those steps seem to be essential, as precipitation-only or filtering-only approaches are reported too. By looking at the amount of material used, sample preparation steps, analysis type, and analysis depth, inventors see differences in the number of identified and quantified intracellular peptides (Table 1) between the published and current invention method. This shows no consensus or accumulated knowledge regarding the efficient and reproducible performance of separating low molecular weight polypeptides from total cells or tissue lysates.

The invention is based on sequencing natural (without modified peptides during the sample preparation and these modifications such as reduction, alkylation, enzymatic digestions, etc.) intracellular peptides of amino acid sequences. However, there are significant differences concerning existing methods, such as in terms of sample preparation, time, and cost-wise, with the proteomics/peptidomics approach [15], [17] for diagnostic and prognostic cardiovascular disease. In Table 1, inventors have compared several limitations of existing methods. These limitations include; complex or multistep sample preparation and the low number of intracellular peptides identification/quantification is the significant limitations in existing methods (Table 1). In the contrary, the invention is high throughput and comprehensive; in total identified 18183 and successfully quantified 16076 intracellular peptides, including the modifications from six cellular samples. Moreover, invention method of is quick (need few hours for two steps sample preparation) and cheaper (do not needs any additional steps like reduction, alkylation, digestion, heating, precipitation, Tandem mass tag (TMT) labeling, etc.) than the existing peptidomics/proteomics method. In addition, it can be applied to mammalian and primary patient-derived cells using an unbiased mass spectrometry tool. The use of intracellular peptidomics for early screening and diagnosis is vital to develop an effective therapy for a wide range of cellular disease models. The additional comparison of previously reported intracellular peptidomics is included in 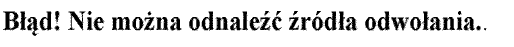

**Table 1. Comparison of source material, sample preparation steps, and qualitative and quantitative peptide numbers between invention method and published methods for intracellular peptides. N/A- nonapplicable, means that the method was not developed for quantitative peptide analysis.**

| Publication | Source material | two-steps sample preparation | Multi-steps sample preparation | Qualitative peptide numbers | Quantitative peptide numbers |
|---|---|---|---|---|---|
| Quantitative Peptidomics of Mouse Brain After Infection With Cyst-Forming *Toxoplasma gondii*[7] | Whole brain | No | Yes | 2735 peptides in 18 runs | 478 and 344 |
| Identification of Non-Canonical Translation Products in *C. elegans* Using Tandem Mass Spectrometry[8] | Caenorh abditis | No | Yes | -500 peptides/ sample | N/A |
| Peptidomics Analysis Reveals Peptide PDCryab 1 Inhibits Doxorubicin-Induced Cardiotoxicity[9] | Whole mouse heart (4hearts/ sample) | No | Yes | 2945 peptides detected | N/A |
| Peptidomic analysis of cultured cardiomyocytes exposed to acute ischemic-hypoxia[10] | 6 million cells/ sample | No | Yes | 4506 in six samples | N/A |
| Comparative peptidome profiling reveals critical roles for peptides in the pathology of pancreatic cancer[5] | Not disclosed | No | Yes | 2881 | N/A |
| Peptidomic Analysis of Endometrial Tissue from Patients with Ovarian Endometriosis[11] | Tissue, not disclosed amount | No | Yes | 491 | N/A |
| Comparison of Human and Murine Enteroendocrine Cells by Transcriptomic and Peptidomic Profiling[12] | Tissue, undefine d or 15-45 mg | No | Yes | Max 1778/sample | N/A |
| Identification of intracellular peptides associated with thermogenesis in human brown adipocytes[13] | 100 million cells/sample | No | Yes | 4370 | N/A |
| Identification and characterization of metformin on peptidomic profiling in human visceral adipocytes[14] | Not disclosed | No | Yes | 3065 | 3065 |
| Proteasome Inhibitors Alter Levels of Intracellular Peptides in HEK293T and SH-SY5Y Cells[6] | 150 mm×25 mm plate/gro up | No | Yes | 558 | N/A |
| Our invention method | 5-10 million cells | Yes | No | at least ≥18183 | at least ≥16076 |

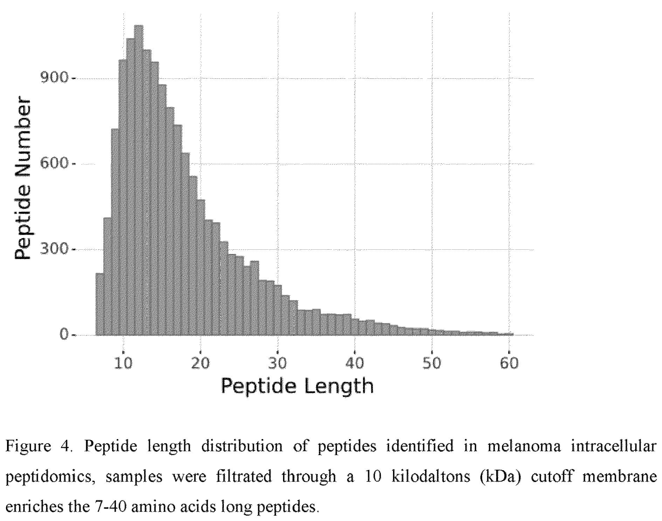

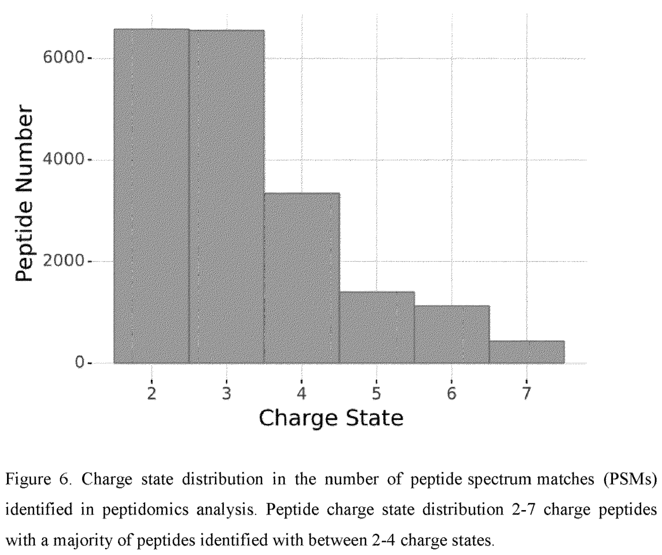

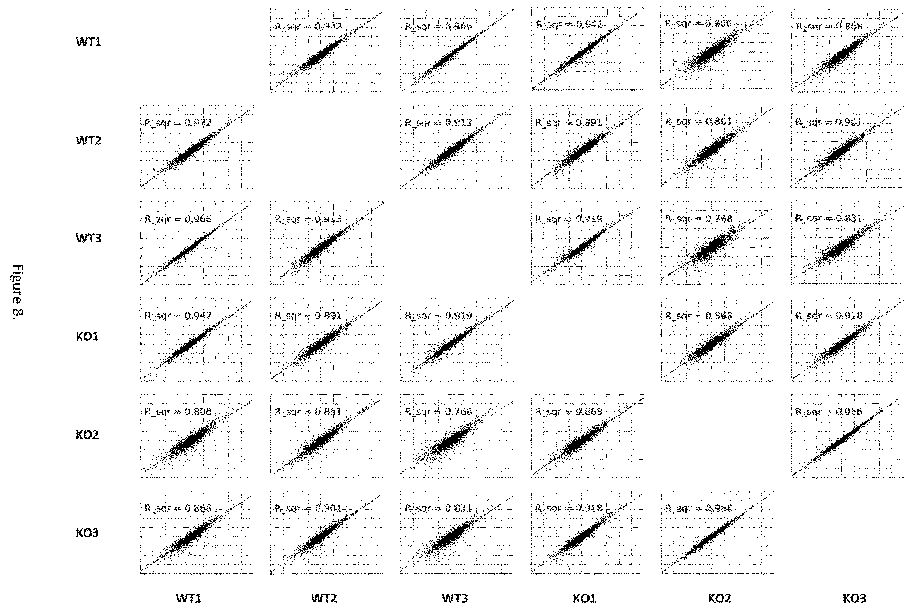

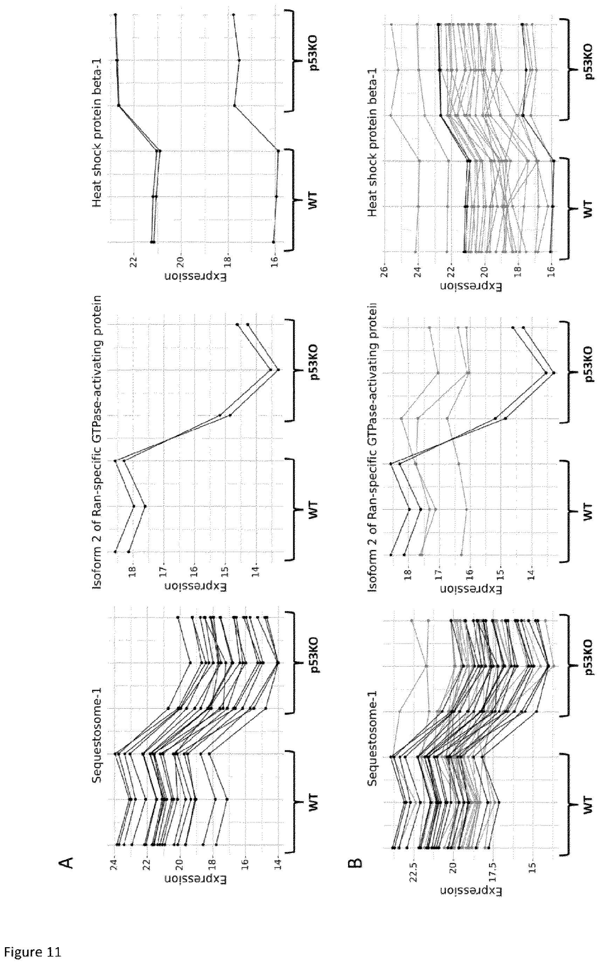

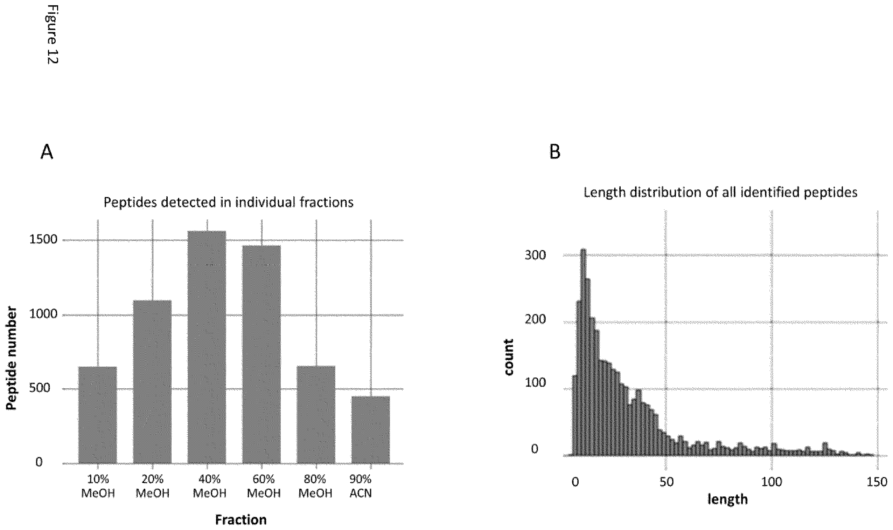

The current invention is simple and differs from those available approaches.
1. Method of sample preparation for qualitative and quantitative analysis of intracellular peptidome in mammalian cells. It is based on amino acid sequencing with a tandem mass spectrometry with signaling intensities measurement of peptides characterized by only two steps of sample preparation.
   a) urea denaturation of mammalian cells, preferably continuous cellular models, primary cells isolated from tissue, and blood samples where a solution of urea (purity ≥99.5%) in phosphate-buffered saline (PBS) without additives (no calcium/magnesium/phenol red) pH 7.2-7.5 is used 20-30 minutes for sonication (ultrasonic bath sonicator without heat) and vortexing at room temperature (RT) to lyse the cells and dissociate protein-peptide interactions;
   b) purification by oasis columns (hydrophilic-lipophilic balanced) for 120-180 minutes at room temperature (RT) to eliminate the abundant proteins and simultaneously elute the cellular peptides further separated with an ultrafiltration approach using selected molecular weight cutoff filter (3 or 10 or 30 kilo daltons, kDa) for 15-30 minutes, 4 - 10 °C and removal of high- molecular weight proteins and other cellular compounds (> 30 kDa). Then liquid chromatography coupled with mass spectrometry (LC/MS) measurement and qualitative, quantitative data analysis.
2. Method of urea (purity ≥99.5%) of 8 M (molar) dissolved in phosphate-buffered saline (PBS) without additives (no calcium/magnesium/phenol red) pH 7.2-7.5 tested and selected for denaturation of intracellular peptides, which has shown superior performance than 200 µl of 50% ACN (acetonitrile) and 50 µl of 100% FA (formic acid) agents.
3. Then the invention method performed sequencing the intracellular peptides based on amino acid sequences with a tandem mass spectrometry followed by intensities signaling measurement in studied melanoma (A375) cellular model in comparison with the matched wild-type (WT) and TP53 knockout (KO) A375 cellular samples.
4. Invention have comprehensive both qualitative and quantitative intracellular peptidomics analysis, wherein based on the comparison with matched wild-type (WT) and TP53 knockout (KO) A375 cell samples identification (qualitative analysis) of at least 18183 intracellular peptides and quantification (quantitative analysis) of at least 16076 intracellular peptides by DIA (data-independent acquisition) 12 run LC-MS/MS (Liquid Chromatography with tandem mass spectrometry) analysis of 6 melanoma cellular samples can be performed.
5. The method can enrich the various sizes according to the molecular weight filters used to collect intracellular peptides. Thus, the filter permeability is not the limitation of the method. Invention have tested 3, 10, and 30 kilodaltons (kDa) filter membranes, each giving a significantly different effect. The summary of peptide-detected intensities with 3, 10, and 30 kilodaltons (kDa) ultrafiltration cutoff membranes demonstrated in figure SA, B.
6. The data analysis workflow for quantitative analysis of the intracellular peptides sequenced with a tandem mass spectrometry obtained according to the following steps within the peptide identification was performed using Fragpipe suite to analyze DDA (data-dependent acquisition), extract and identify pseudo-DDA spectra from DIA (data-independent acquisition) runs and create spectral library compatible with further analysis. DIA quantification was performed by DIA-NN using spectral libraries created in the previous step. DIA-NN was also used to perform automatic data normalization. Finally, further processing was done with Perseus or custom Python scripts.
7. The quantitative analysis of the intracellular peptides sequenced with tandem mass spectrometry can be performed when signal intensity of the studied peptide is at t-test and permutation-based false discovery rate (FDR) correction at 1% FDR (false discovery rate) between wild-type (WT) and TP53 knockout (KO) A375 cell samples. Invention quantitative data shows, namely, Sequestosome-1 (SQSTM1), Ran-specific GTPase-activating protein (RANG), and Heat shock protein beta-1 (HSPB1) are sources of subsets of differentially abundant peptides detected in melanoma A375 TP53 knockout (KO) compared with wild type (WT) experiment (figure 11a). In addition, the intracellular peptidomics approach discriminates the differentially regulated and non-regulated peptides even though the source of the protein is the same (figure 11b).
8. The method highlights several crucial features of intracellular peptidomics such as a) peptide length distribution, while using 10 kilodaltons (kDa) cutoff membrane enriches the 7-40 amino acids long peptides (figure 4), and by multiple elution/fractionation, the wider length distribution of intracellular peptides from 7 to 130 amino acids long identified in all fractions (figure 12B), b) peptide charge state distribution 2-7 charge peptides with a majority of peptides identified with between 2-4 charge states (figure 6), c) distribution of valid value numbers on precursor level from quantified peptides, in the invention most of the peptides quantified with valid values = 6, which shows invention has highly confident quantification qualities, d) peptide-level quantification reproducibility (scatterplots of biological replicate (WT and KO) measurements with Pearson R² correlation coefficient show are higher than 0.8 and 0.9 (0.8 and 0.9 > R² ) which show high level of correlation for peptide level quantification, a good level of reproducibility between (figure 8) of intracellular peptidomics from the cellular model.

The invention has the following advantages:
1. Major difference is focusing on intracellular peptides and not proteins.
2. The existing approaches were developed without depletion and precipitation steps, allowing us to yield more peptides and isolate peptides possibly bound to highly abundant proteins. The invention is fast/quick (needs a few hours for two steps sample preparation) and cost-effective/cheaper (does not needs any additional steps like reduction, alkylation, digestion, heating, precipitation, Tandem mass tag (TMT) labeling, etc.) than the existing peptidomics/proteomics method. In addition, it can be applied to mammalian and primary patient cells using an unbiased mass spectrometry tool.
3. The invention does not need to use additional purification or desalting kits (Ettan 2D clean) [17], [18]; all these steps are time and cost-consuming.
4. For high sensitivity, a known method [17] used fluorescent dye (Cy3, 5) for protein labeling. Reading and imaging the proteome profile from the dye-based gel needs a specific instrument (The Typhoon instrument, GE Healthcare variable-mode imager that produces digital images of radioactive, fluorescent, or chemiluminescent samples), which again needs an investment of time and money. Therefore, altogether, it is a complex sample preparation procedure and needs an experienced person to perform these kinds of analyses.
5. The invention is completely free from additional manipulation steps such as reduction, alkylation, and enzymatic (tryptic) digestion. It also allows identifying natural, endogenous peptides (which can serve as unique markers) without any modifications. The method is simple and has only two steps sample preparation, a) urea denaturation method, allowing cell lysis and the dissociation of protein-peptide interactions, b) purification by oasis columns (hydrophilic-lipophilic balanced) to eliminate the abundant proteins and simultaneously elute the cellular peptides further separated with an ultrafiltration approach using molecular weight cutoff (MWCO) filters (3/10/30 kilodaltons, kDa) and for removal of high-MW (molecular weight) compounds. While simple, this method removes sufficient interference to enable peptide detection without compromising quantitative reproducibility.
6. The Known method shows that they only focus on identifying proteins/peptides. However, the invention is based on performance on both qualitative and quantitative analysis. The invention is comprehensive for both qualitative and quantitative intracellular peptidomics analysis, wherein based on the comparison with matched wild-type (WT) and TP53 knockout (KO) A375 cell samples identification (qualitative analysis) of at least 18183 intracellular peptides and quantification (quantitative analysis) of at least 16076 intracellular peptides by DIA (data-independent acquisition) 12 run LC-MS/MS (Liquid Chromatography with tandem mass spectrometry) analysis of 6 melanoma cellular samples can be performed.
7. The instrumentation (most advanced mass spectrometry model), previously, for the separation and identification of peptides, the inventors used two-dimensional difference gel electrophoresis (2D DIGE), Surface-enhanced laser desorption/ionization (SELDI) or modified liquid chromatography-Matrix-Assisted Laser Desorption/Ionization mass spectrometry (LC-MALDI), Matrix-Assisted Laser Desorption/Ionization-Time Of Flight mass spectrometry MALDI TOF/MS based platforms for various biological samples. These platforms are less sensitive and comprehensive than Exploris 480 orbitrap-based mass spectrometer. In short, the inventors have developed a novel, without precipitation, protein labeling by fluorescent dye, gel electrophoresis, desalting etc., and a direct qualitative, quantitative, comprehensive intracellular peptidomics approach.
8. Discovery experiments: recently emerged field of research concentrate of Small Open Reading Frames (sORFs), a subset of very short proteins (10-100 amino acids) that were previously ignored in proteomic databases and, therefore, remained largely undetected. In this field, especially in discovery-oriented experiments, sample preparation is concentrated on separating those small polypeptides from other cellular components, especially larger proteins. Still, subsequent steps usually involve enzymatic digestion to improve the identification of larger peptides; however, additional or exclusive top-down analysis of intact polypeptides is possible due to their relatively small size. Inventors have spotted several highly successful examples of peptidome analyses, proving that this field may enhance knowledge about cellular physiology and provide new directions for applied research. Quantitative peptide analysis and identification extended to non-canonical sequences identified based on genomic and transcriptomic data make it possible to propose. Furthermore, peptidomics is a valuable tool for studying protein degradation (cit proteasome) as it provides a chance to obtain an overall landscape of protein degradation products present in the cells. Until recently, research on the intracellular peptidome and micro proteome (fraction of proteins with very low molecular weight) was complex, among others. Therefore, the Invention of this methodology of intracellular peptidomics can be used complementary in a wide range of basic and applied research. Further development of peptidomics as a method of identifying novel biologically relevant polypeptides and biomarker discovery has been applied in different cellular models.

The invention is considered a highly sensitive, reproducible, label-free liquid chromatography-mass spectrometry (LC-MS/MS) approach on wild-type (WT) and TP53 knockout (KO) A375 cell models to find the TP53 -dependent variation in intracellular peptidome. Hence, the invention cellular model system and its peptidomics data are complementary to other -omics techniques, such as proteomics and immunopeptidomics. The invention is readily applicable worldwide based on *in vitro* cellular models. Cell culture is one of the finest tools for basic science research; each research lab has this facility. Moreover, it is easy to grow the cells or produce specific cell models; knockout genes or silencing the genes in the cellular model depends on the biological questions. Researchers do not need additional ethical approval (while working with and conducting any animal need ethical approval) studies to work with cells. Therefore, cell-based models are easily and readily available for basic science research at university-level standard laboratories. In addition, the invention approach is comparatively faster (time-wise) and cheaper for directly finding significant biological intracellular peptides from the cell culture-based model using the unbiased mass spectrometry analytical tool.
These proteomics/peptidomics samples preparation have completely different sample preparation steps, such as precipitation, protein labeling by fluorescent dye, gel electrophoresis, trypsin digestion, desalting, etc., from the invention peptidomics approach. Whereas in invention approach bypasses all these time and cost-consuming steps. On the contrary, the invention enables us to find several hundred/thousands of naturally occurring peptides from different cellular conditions (wild type/WT and TP53 KO gene melanoma cellular model). Moreover, this is a fantastic opportunity to bring these outcomes to the clinical level or develop strategies that can be employed directly to animal models, patients, or clinical samples.

Therefore, the invention method of sample preparation for qualitative and quantitative analysis of intracellular peptidome in mammalian cells. It is based on amino acid sequencing with tandem mass spectrometry with signaling intensities measurement of peptides, characterized in that the method comprises two steps of sample preparation as follows:
1a) urea denaturation of the sample of mammalian cells, preferably continuous cellular models, primary cells isolated from tissue, and blood samples, where a solution of urea (purity ≥99.5%) in phosphate-buffered saline (PBS) pH 7.2-7.5 is used for 20-30 minutes for sonication and vortexing at room temperature to lyse the cells and dissociate protein-peptide interactions;
1b) purification by oasis columns (hydrophilic-lipophilic balanced) for 120-180 minutes at room temperature to eliminate the abundant proteins and simultaneously elute the cellular peptides further separated with an ultrafiltration approach using selected molecular weight cutoff filter that has 3-30 kilo daltons kDa, preferably 3 or 10 or 30, kDa for 15-30 minutes at 4 - 10 °C and removal of high- molecular weight proteins and other cellular compounds that have > 30 kDa;
2. Method comprises the following steps:
   a) urea denaturation of mammalian cells, preferably continuous cellular models, primary cells isolated from tissue, and blood samples where a solution of urea (purity ≥99.5%) in phosphate-buffered saline (PBS) without additives (no calcium/magnesium/phenol red) pH 7.2-7.5 is used 20-30 minutes for sonication (ultrasonic bath sonicator without heat) and vortexing at room temperature (RT) to lyse the cells and dissociate protein-peptide interactions;
   b) purification by oasis columns (hydrophilic-lipophilic balanced) for 120-180 minutes at room temperature (RT) to eliminate the abundant proteins and simultaneously elute the cellular peptides further separated with an ultrafiltration approach using selected molecular weight cutoff filter of 3 or 10 or 30 kilo daltons, kDa for 15-30 minutes, 4 - 10 °C and removal of high- molecular weight proteins and other cellular compounds of > 30 kDa). Then peptides are subjected to the liquid chromatography coupled with mass spectrometry (LC/MS) measurement and qualitative, quantitative data analysis.
3. Method wherein urea (purity ≥99.5%) of 8 M dissolved in phosphate-buffered saline (PBS) without additives (no calcium/magnesium/phenol red) pH 7.2-7.5 is used for denaturation of intracellular peptides.
4. The method as the sample the mammalian cells sample is used.

### Essence of the invention - description of the invention

Method for high-throughput qualitative and quantitative intracellular peptidomics of mammalian cells.

The goal of the invention was to develop a method of sample preparation for comprehensive qualitative and quantitative analysis of intracellular peptidome in mammalian cellular models (e.g., human melanoma) based on amino acid sequencing with a tandem mass spectrometry (MS) tool with signaling intensities measurement of peptides. This invention method can apply to all types of cells model, including floating and adherent cells, immune cells, cells from cultures *in vitro,* or freshly isolated primary cells from clinical samples. This is a fast method; It has only two steps of sample preparation 1) denaturation step, allowing denaturation of cellular peptides and the dissociation of protein-peptide interactions. 2) purification of isolated intracellular peptides is performed by using hydrophilic-lipophilic balanced columns that lead to the removing of proteins and peptides equal or bigger than 30kDa from peptides enriched and simultaneously purification of peptides using hydrophilic-lipophilic balanced columns to obtain peptides smaller than 30 kDa. Therefore, the invention does not comprise any additional step for highly concentrated protein and peptides depletion and precipitation, which ultimately improves the final yield of intracellular peptides and prevents the losses of the many biomarker intracellular peptides during depletion and precipitation steps. Moreover, it simultaneously elutes the intracellular peptides further separated with an ultrafiltration approach using molecular weight cutoff (MWCO) filters (3/10/30 kilodaltons, kDa) and for removal of high-MW (high- molecular weight) protein and other cellular compounds. While simple, this method removes sufficient interference to enable peptide detection without compromising quantitative reproducibility. Then the MS (Mass Spectrometry) measurement and data analysis. The invention includes a unique sample preparation approach for qualitative and quantitative analysis of intracellular peptidome. Particular emphasis was placed on the optimization of the denaturation step, purification, chromatographic separation, identification, and quantification of native cell peptides using liquid chromatography coupled with mass spectrometry (LC/MS).

Therefore, the invention includes the following key highlights;
1. Method of sample preparation for qualitative and quantitative analysis of intracellular peptidome in mammalian cells based on amino acid sequencing with tandem mass spectrometry with signaling intensities measurement of peptides characterized by the method only two steps of sample preparation.
   a) urea denaturation of mammalian cells, preferably continuous cellular models, primary cells isolated from tissue, and blood samples where a solution of urea (purity ≥99.5%) in phosphate-buffered saline (PBS) without additives (no calcium/magnesium/phenol red) pH 7.2-7.5 is used 20-30 minutes for sonication (ultrasonic bath sonicator without heat) and vortexing at room temperature (RT) to lyse the cells and dissociate protein-peptide interactions;
   b) purification by oasis columns (hydrophilic-lipophilic balanced) for 120-180 minutes at room temperature (RT) to eliminate the abundant proteins and simultaneously elute the cellular peptides further separated with an ultrafiltration approach using selected molecular weight cutoff filter (3 or 10 or 30 kilo daltons, kDa) for 15-30 minutes, 4 - 10 °C and removal of high- molecular weight proteins and other cellular compounds (> 30 kDa). Then peptides are subjected to the liquid chromatography coupled with mass spectrometry (LC/MS) measurement and qualitative, quantitative data analysis.
2. Method of urea (purity ≥99.5%) of 8 M (molar) dissolved in phosphate-buffered saline (PBS) without additives (no calcium/magnesium/phenol red) pH 7.2-7.5 tested and selected for denaturation of intracellular peptides, which has shown superior performance than 200 µl of 50% ACN (acetonitrile) and 50 µl of 100% FA (formic acid) agents.
3. The intracellular peptides are obtained in various sizes according to the molecular weight filters (3 or 10 or 30 kilo daltons, kDa) used to collect intracellular peptides.
4. The invention method can be applied to all mammalian cells. preferably continuous cellular models, primary cells isolated from tissue, and blood samples.
5. In the invention, additional 2-6 elutions/fractionations can be used to enrich of longer (7 - 130 amino acids) size of intracellular peptides.

The invention is described precisely in the example and drawing in where the following information is shown: figure 1. the workflow of intracellular peptidomics sample preparation is shown, figure 2. development of denaturation step for qualitative analysis comparing identified intracellular peptides, figure 3. venn diagram of qualitative analysis comparing identified peptides and checked the performance of developed 3a. DDA (data-dependent acquisition) and 3b. DIA (data-independent acquisition) spectrometry-based peptidomics research methods in all peptidome samples, figure 4. peptide length distribution of intracellular peptides identified in melanoma peptidome, samples were filtrated through a 10 kilodaltons (kDa) cutoff membrane, figure 5A. size distribution of polypeptide ions detected in samples prepared using various filter sizes. 5B. summed intensities of all polypeptide ions detected in samples prepared using various filter sizes, figure 6. charge state distribution in the number of peptide spectrum matches (PSMs) identified in peptidomics analysis, figure 7. distribution valid value number on precursor level from quantified peptides, figure 8. the peptide level quantification reproducibility between the cellular samples, figure 9. shown the peptide volcano plot shows (as dark dots) dysregulated intracellular peptides with adj. p-value < 0.01 in comparisons of wild type (WT) to TP53 knockout (KO) A375 cells, figure 10. shown string network analyses of identified sources of peptides with statistically different abundances in TP53 knockout/KO A375 melanoma cells, figure 11. quantitative peptide abundance profiles for selected source proteins between two groups (wild type/WT and TP53 knockout/KO), exhibiting a substantial share of 11a. regulated and 11b. non-regulated peptides, figure 12A. number of peptides detected in each fraction. 12B. length distribution of peptides identified in all fractions.

### Detailed description of figures:

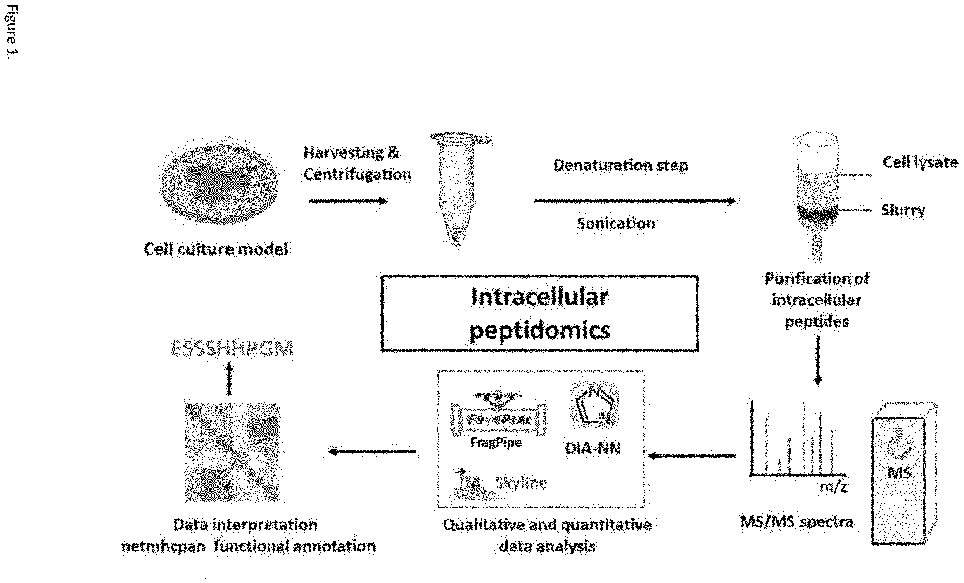

Figure 1. Workflow of intracellular peptidomics sample preparation, two steps intracellular peptidomics sample preparation includes, first step is to use denaturation step, allowing cell lysis and the dissociation of protein-peptide interactions. The second key step is purification by oasis columns (hydrophilic-lipophilic balanced) to eliminate the abundant proteins and simultaneously elute the cellular peptides further separated with an ultrafiltration using 10 kilodaltons (10 kDa) molecular weight cutoff (MWCO) filters and for removal of high-MW proteins and other cellular compounds. While simple, this method removes sufficient interference to enable peptide detection without compromising quantitative reproducibility.

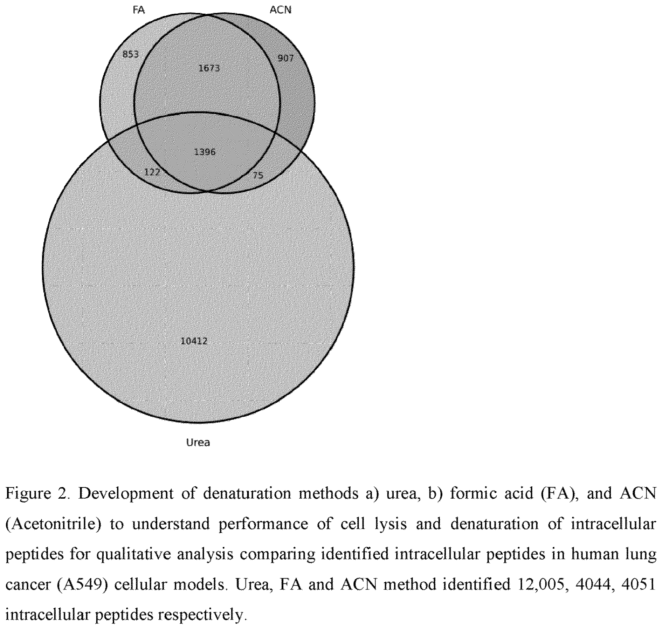

Figure 2. Development of denaturation methods a) urea, b) formic acid (FA), and ACN (Acetonitrile) to understand the performance of cell lysis and denaturation of intracellular peptides for qualitative analysis comparing identified intracellular peptides in human lung cancer (A549) cellular models. Urea, FA and ACN methods identified 12,005, 4044, and 4051 intracellular peptides, respectively.

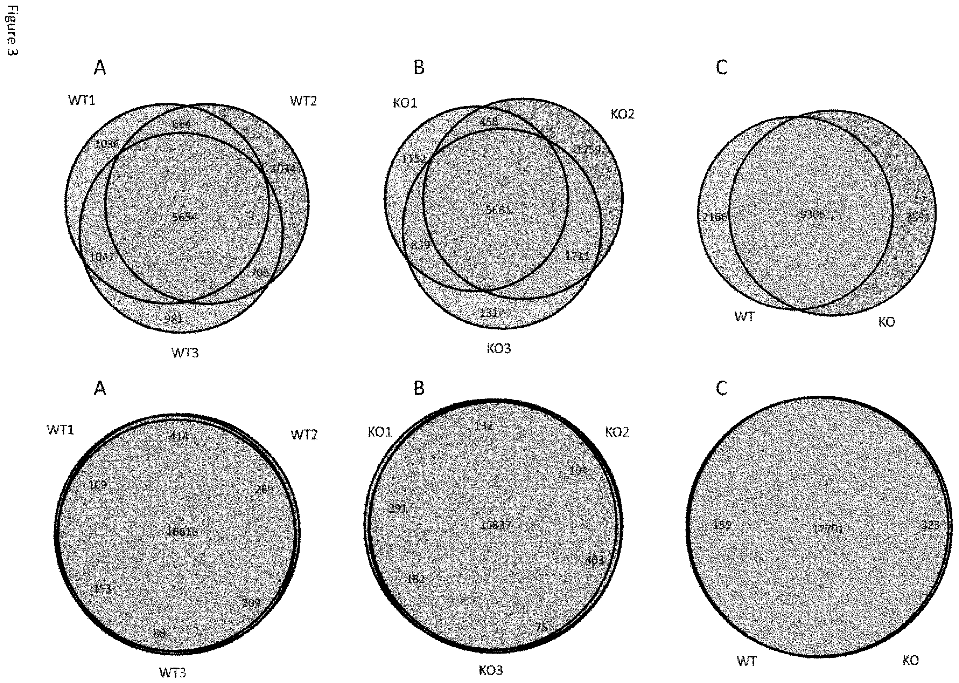

Figure 3a.Venn diagram of qualitative analysis comparing identified peptides in DDA (data-dependent acquisition) A) biological replicates of wild type (WT) cells, B) biological replicates of TP53 knockout (KO) cells, C) two conditions (wild type/WT vs TP53 knockout/KO, all replicates combined for each condition).
Figure 3b.Venn diagram of quantitative analysis comparing peptides quantified in DIA (data-independent acquisition) A) biological replicates of wild type (WT) cells, B) biological replicates of p53 knockout (KO) cells, C) two conditions (wild type/WT vs TP53 knockout/KO, all replicates combined for each condition). As visible here, vast majority of peptides are uniformly quantified in all replicates. This present the very high quantification reproducibility and coverage offered by DIA (data-independent acquisition) based peptidomics analysis.
Figure 4. Peptide length distribution of peptides identified in melanoma intracellular peptidomics, samples were filtrated through a 10 kilodaltons (kDa) cutoff membrane that enriches the 7-40 amino acids long peptides.

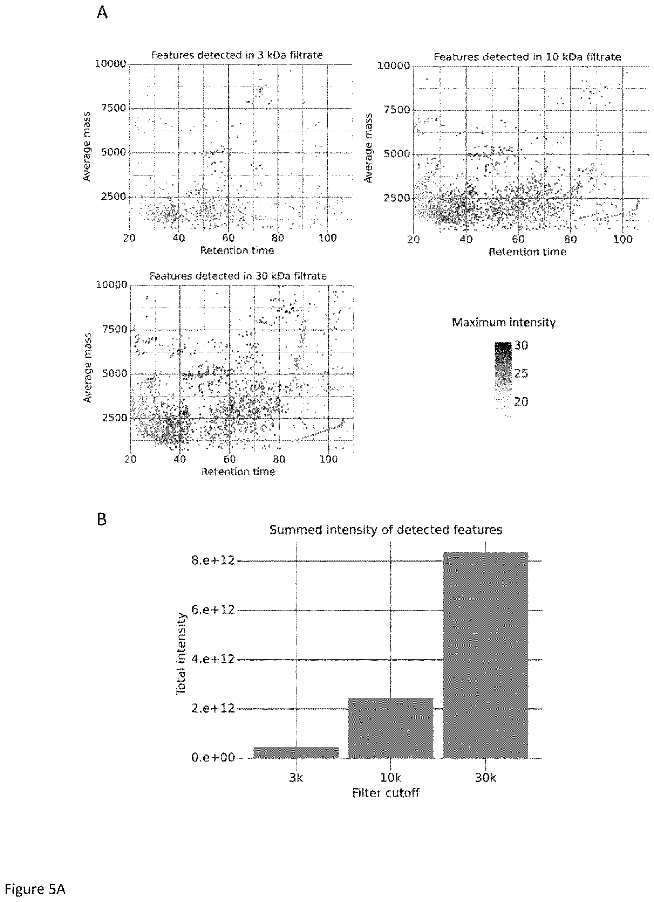

Figure 5A. Size distribution of polypeptide ions detected in samples prepared using various filter sizes. 5B. Summed intensities of all polypeptide ions detected in samples prepared using various filter sizes (3, 10, and 30 kilodaltons).
Figure 6. Charge state distribution in the number of peptide spectrum matches (PSMs) identified in peptidomics analysis. Peptide charge state distribution 2-7 charge peptides with a majority of peptides identified with between 2-4 charge states.

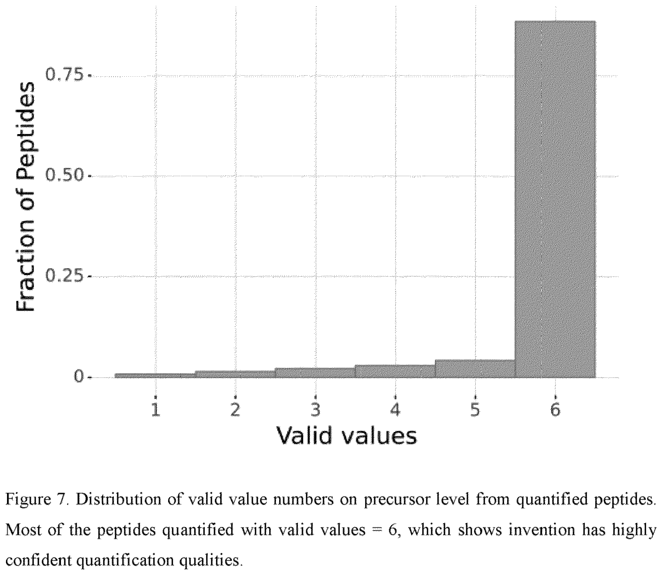

Figure 7. Distribution of valid value numbers on precursor level from quantified peptides. Most of the peptides were quantified with valid values = 6, which shows the invention has highly confident quantification qualities.
Figure 8. Peptide level quantification reproducibility between the cellular samples. Scatterplots of biological replicate measurements with Pearson R2 correlation coefficient are higher than 0.8 and 0.9 (0.8 and 0.9 > R2 ), which show a high level of correlation for peptide level quantification, a good level of reproducibility between samples (biological replicated of WT and KO cellular samples).

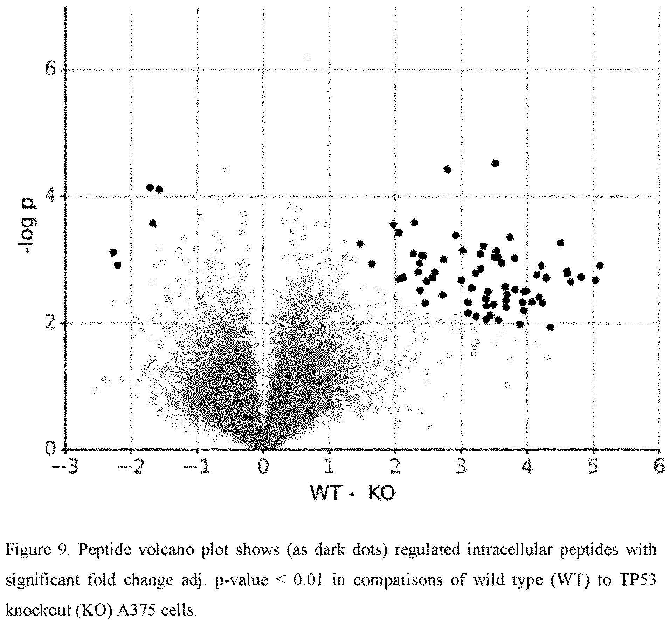

Figure 9. Peptide volcano plot shows (as dark dots) regulated intracellular peptides with significant fold change adj. p-value < 0.01 in comparisons of wild type (WT) to TP53 knockout (KO) A375 cells.

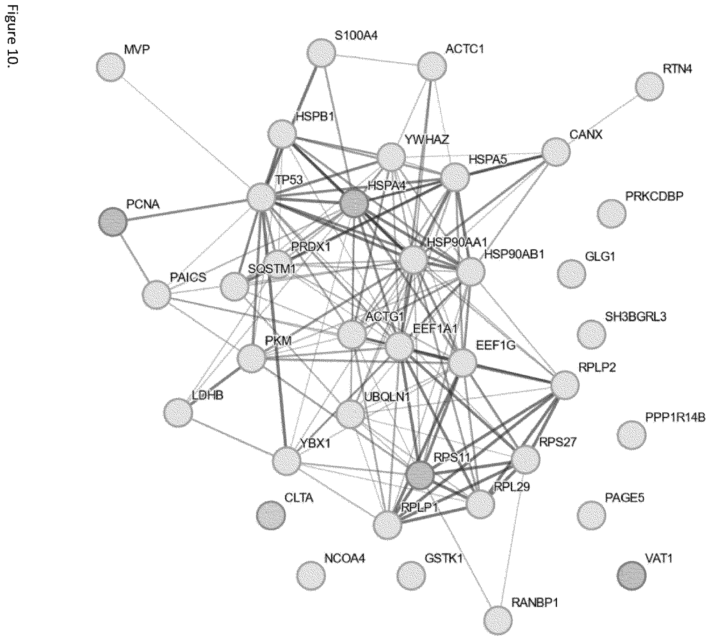

Figure 10. String network analyses of identified sources of peptides with statistically different abundances in TP53 knockout A375 melanoma cells. The putative interaction network shown above suggests high functional connectivity between obtained hits.
Figure 11a. Quantitative peptide abundance profiles for selected source proteins, exhibiting substantial share of regulated peptides between wild type (WT) and p53 KO A375 melanoma cells.
Figure 11b. Quantitative peptide abundance profiles for selected source proteins, exhibiting substantial share of regulated and non-regulated peptides between wild type (WT) and p53 KO A375 melanoma cells.
Figure 12A. Number of peptides detected in each fraction. for fractionations eluted in six fractions using 10%, 20%, 40%, 60%, and 80% of methanol in 0.2% formic acid volume per volume (v/v) (in this solution, final proportion of methanol and formic acid in water was 10%, 20%, 40%, 60%, 80%, and 0.2%, respectively) and 90% acetonitrile in 1% formic acid volume per volume (v/v) (in this solution the final proportion of acetonitrile and formic acid in water was 90% and 1%, respectively). 12B. Length distribution (7 to 130 amino acids long) of peptides identified in all fractions.

Method of sample preparation for qualitative and quantitative analysis of intracellular peptidome in mammalian cells. It is based on amino acid sequencing with tandem mass spectrometry with signaling intensities measurement of peptides characterized. The method comprises only two steps of sample preparation.
1a) urea denaturation of mammalian cells, preferably continuous cellular models, primary cells isolated from tissue, and blood samples where a solution of urea (purity ≥99.5%) in phosphate-buffered saline (PBS) without additives (no calcium/magnesium/phenol red) pH 7.2-7.5 is used 20-30 minutes for sonication (ultrasonic bath sonicator without heat) and vortexing at room temperature (RT) to lyse the cells and dissociate protein-peptide interactions;
1b) purification by oasis columns (hydrophilic-lipophilic balanced) for 120-180 minutes at room temperature (RT) to eliminate the abundant proteins and simultaneously elute the cellular peptides further separated with an ultrafiltration approach using selected molecular weight cutoff filter (3 or 10 or 30 kilo daltons, kDa) for 15-30 minutes, 4 - 10 °C and removal of high- molecular weight proteins and other cellular compounds (> 30 kDa). Then peptides are subjected to the liquid chromatography coupled with mass spectrometry (LC/MS) measurement and qualitative, quantitative data analysis.
2. Method of urea (purity ≥99.5%) of 8 M (molar) dissolved in phosphate-buffered saline (PBS) without additives (no calcium/magnesium/phenol red) pH 7.2-7.5 tested and selected for denaturation of intracellular peptides, which has shown superior performance than 200 µl of 50% ACN (acetonitrile) and 50 µl of 100% FA (formic acid) agents.
3. The intracellular peptides are obtained in various sizes according to the molecular weight filters (3 or 10 or 30 kilo daltons, kDa) used to collect intracellular peptides. Thus, the filter permeability is not the limitation of the method. The invention method can be applied to all mammalian cells. Invention have comprehensive both qualitative and quantitative intracellular peptidomics analysis, wherein based on the comparison with control cell samples identification (qualitative analysis) of at least 18183 intracellular peptides and quantification (quantitative analysis) of at least 16076 intracellular peptides by LC-MS/MS (Liquid Chromatography with tandem mass spectrometry) analysis.

The method highlights several crucial features of intracellular peptidomics, such as a) peptide length distribution, while using 10 kilodaltons (kDa) cutoff membrane enriches the 7-40 amino acids long peptides (figure 4). In addition, fractionations facilitate enriching a wider length distribution of intracellular peptides from 7 to 130 amino acids long identified in all the fractions (figure 12B). The longer (7 to 130 amino acids) and without enzymatically digested intracellular peptides identification allows to screen disease-specific peptides which are directly applicable for understanding the basic biological mechanisms and biomarker discovery, b) peptide charge state distribution 2-7 charge peptides with a majority of peptides identified with between 2-4 charge states (figure 6), c) distribution of valid value numbers on precursor level from quantified peptides, in the invention most of the peptides quantified with valid values = 6, which shows invention has highly confident quantification qualities, d) peptide-level quantification reproducibility (scatterplots of biological replicate (WT and KO) measurements with Pearson R² correlation coefficient show are higher than 0.8 and 0.9 (0.8 and 0.9 > R² ) which show high level of correlation for peptide level quantification, a good level of reproducibility between (figure 8) of intracellular peptidomics from the cellular model.

### Detailed sample preparation and analysis methods are applied in the following examples

### Example 1

The method is novel, simple, and it has only two steps in sample preparation and at least one additional step (figure 1). Prior to starting any steps of the intracellular peptidomics method, it is strongly recommended to prepare all the solutions using LC-MS (Liquid Chromatography with mass spectrometry) grade water, solvents, reagents, and ultraclean glass and plasticware use during methods. Moreover, all solutions, and buffers should be prepared immediately before use and disposed of after one week of storage. Cellular samples should be collected directly from the cell culture laboratory, subjected to the washing with phosphate-buffered saline (PBS) and centrifuged at 3000 ×g for 10 min at 4 °C. The cellular pellet can be stored at -80 °C until analysis. The first step is to prepare all the necessary buffers on the same day of peptidomics samples preparation.

### Buffer preparations

the 1^{st} solution, urea denaturation step: 12 gram of urea (purity ≥99.5%) were weighted to achieve 8 Molar concentration in 25ml, and dissolved in phosphate-buffered saline (PBS) without additives (no calcium/magnesium/phenol red) pH 7.2-7.5 (Gibco PBS tablets) added to 25ml volume.
2^{nd} solution, 0.2% formic acid/methanol volume per volume (v/v) was prepared (final proportion of formic acid in methanol was 0.2%).
3^{rd} solution, 0.2% formic acid/water volume per volume (v/v) was prepared (final proportion of formic acid in water was 0.2%).
4^{th} solution wash buffer, water/5% methanol/0.2% formic acid volume per volume (v/v) was prepared (the final proportion of methanol and formic acid in water was 5% and 0.2%, respectively).
5^{th} solution elution buffer, water/80% methanol/0.2% formic acid volume per volume (v/v) was prepared (the final proportion of methanol and formic acid in water was 80% and 0.2%, respectively).

### Peptides denaturation step, as it is shown in figure 1

Efforts were made to develop a repeatable and effective method to obtain a sufficient amount of sample from the smallest possible amount of input cellular material. The pellet of 5-10 million melanoma cells from each biological replicate was mixed with urea (purity ≥99.5%) denaturation method by vigorous pipetting. After adding the denaturation agent, the first step was sonication (ultrasonic bath sonicator without heat) for 30 minutes at room temperature. Further, cellular samples were vortexed at maximum speed for 30 minutes at room temperature. Then, lysates were centrifuged at 17000 ×g for 15 minutes at room temperature to remove insolubilized cellular debris. The supernatant was collected and diluted to 6ml with 0,2% formic acid (FA) in LC-MS (Liquid Chromatography with mass spectrometry) water (v/v). Lastly, the diluted lysates were centrifuged for 10 min at 5000 ×g to remove residual debris immediately before loading on hydrophilic-lipophilic balanced columns.

### Purification of intracellular peptides, as it is shown in figure 1

All the diluted samples (∼ 6ml each) were subjected to the Oasis cartridges (30 mg; Waters, hydrophilic-lipophilic balanced columns) to perform reverse-phase purification of polypeptides. All steps were performed at room temperature using only gravity flow, and all the detailed steps are as follows.
i) Cartridge's condition: cartridge was conditioned with 0.2% formic acid/methanol volume per volume (v/v) (in this solution final proportion of formic acid in methanol was 0.2%), twice, volume for each wash was 1ml/cartridge.
ii) Equilibration of cartridge: the cartridge was equilibrated with 0.2% formic acid/water volume per volume (v/v) (in this solution final proportion of formic acid in water was 0.2%), volume for each wash was 1ml/cartridge.
iii) Sample loading: 6 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) was eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively),

All filtration unit conditioning and filtration steps were performed immediately after elution. According to the instructions, the mixture was spun through 10 kilodaltons (kDa), molecular weight cutoff ultrafiltration devices (Millipore). Briefly, rinse Amicon-10kDa centrifugal filter before use (rinse the device with LC-MS (Liquid Chromatography with mass spectrometry) grade water, 0.5ml 40% methanol in water/column, and centrifuge at 14000 × g, 30 min, 4 °C). Next, insert Amicon-10kDa centrifugal filter/device into the new collecting tube. Lastly, the pipette eluted and diluted peptide solution (2ml) into the device, taking care not to touch the membrane with the pipette tip. Spin at 14000 × g, 15 min, 4 °C. Collect the filtrate (peptides), then evaporate it to dryness. All dried peptide samples were stored at -80 until the mass spectrometry analysis.

### Example 2

As it is shown in figure 2, initially, inventors used lung cancer (A549) cellular models to determine the best denaturation method. Inventors considered three denaturation agents, a) urea, b) formic acid (FA), and ACN (Acetonitrile) based denaturation of intracellular peptides to understand the performance of cell lysis. 5-10 million human lung cancer cells (A549) were denaturation using above mentioned three different denaturation agents:
- 1 ml of 8M (molar) urea (purity ≥99.5%) in phosphate-buffered saline (PBS) without additives (no calcium/magnesium/phenol red) pH 7.2-7.5
- 200 µl of 50% ACN (acetonitrile)
- 50 µl of 100% FA (formic acid)

All samples were diluted to 6ml with 0.2% FA in water. Based on qualitative results, the overlap of peptides was identified using three different cell denaturation methods shown in figure 2. The venn diagram (figure 2) is to justify that it has appropriate mass cellular peptide isolation protocol and mass spectrometry-based peptidomics acquisition methods, DIA (data-independent acquisition) and DDA (data-dependent acquisition).
Peptides were identified by FragPipe, searching against the human UniProt database with 7-60AA (amino acids) length range, allowing methionine oxidation and peptide N-term acetylation as variable modifications. Urea, FA (formic acid), and ACN (acetonitrile) method identified 12,005, 4044, 4051 intracellular peptides, respectively. Venn diagram (figure 2) allows us to see the benefit of using the urea denaturation step than other methods. While combining the number of identified intracellular peptides between three approaches, the urea based denaturation method was highly superior in terms of overall efficiency, however two other approaches show that they extract distinct sets of unique peptides. Hence, the urea denaturation method allows it to work comprehensively and given higher peptides yield so inventors have selected urea based denaturation approach for melanoma cell model experiments. Remarkably, 5-10 million of A375 melanoma cells for each condition. The two groups were considered, namely A375 wild type (WT) and TP53 knockout (KO) melanoma cells. A biological triplicate of each 5-10 million human melanoma cells (A375) from wild-type (WT) and TP53 knockout (KO) were lysed using a urea denaturation agent.

### Example 3

Mass spectrometry combined with chromatographic separation is necessary to identify more than a few peptides. Due to the unspecific nature of endogenous peptides, successful identification requires order of magnitude higher data quality than a standard trypsin-based proteomic sample, especially in terms of mass accuracy. Only recently, technological advances in mass spectrometry enabled the collection of data of quality acceptable for such analyses. DIA - (data-independent acquisition) method enables to collection of quantitative data for all peptides present in the sample while maintaining good resilience to matrix interferences.

### LC-MS/MS (Liquid Chromatography with tandem mass spectrometry) analysis

### a) LC/MS for denaturation method comparison

Before analysis, samples were dried on speedvac and resuspended in 40 µl of 2,5% acetonitrile and 0,08% trifluoroacetic acid in water. Samples were separated on Thermo Scientific RSLC3000nano LC system, coupled to Thermo Scientific Orbitrap Exploris 480 mass spectrometer. Chromatography was performed by concentrating peptides on 0.3x5mm C18 PepMap trap column (Thermo Scientific) at flow of 5µl/min for 10min and further separation on 0.075mm × 250mm 2um C18 PepMap RSLC column (Thermo Scientific). Loading buffer was identical to the sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 40% in 90 minutes, followed with washing with 99% B and re-equilibration to initial conditions.
Two runs in DDA (data-dependent acquisition) mode per sample were performed with different parameters.

First run, aimed at the identification of relatively short peptides used full scan with 120k resolution, 350-1200 Th mass range, 300% AGC (automatic gain control) target and automatic maximum injection time. Full scans were saved as profile spectra. For fragmentation, ions of charge state 2 to 6 and minimum intensity of 5000 were selected. Dynamic exclusion was set to 20s within +/-10ppm (parts per million) range from fragmented precursor and its isotopes. Fragmentation settings were as follows. Ion isolation window was set to 2 Th, resolution to 60k and normalized HCD (higher energy collisional dissociation) fragmentation energy to 30%. AGC (automatic gain control) target was set to standard, and scan range and maximum ion injection time to Auto. Fragmentation scans were saved as centroided spectra.

The second run, aimed at the identification of a wide mass range of peptides, used full scan with 120k resolution, 350-1800 Th mass range, 300% AGC (automatic gain control) target and automatic maximum injection time. Full scans were saved as profile spectra. For fragmentation minimum intensity of 5000 were selected and dynamic exclusion was set to 20s within +/-10ppm (parts per million) range from fragmented precursor ant its isotopes. Fragmentation settings were dependent on ion charge. For 2 to 5 charge state ion isolation window was set to 2 Th, resolution to 30k and normalized HCD (higher energy collisional dissociation) fragmentation energy to 30%. AGC (automatic gain control) target was set to standard, and scan range and maximum ion injection time to Auto. For 6 to 10 charge state ion isolation window was set to 2 Th, resolution to 60k and normalized HCD (higher energy collisional dissociation) fragmentation energy to 30%. AGC (automatic gain control) target was set to 300%, and scan range and maximum ion injection time to Auto. Fragmentation scans were saved as centroided spectra.

### b) LC/MS (Liquid Chromatography with tandem mass spectrometry) for ultrafiltering comparison

Before analysis, samples were dried on speedvac and resuspended in 40 µl of 2,5% acetonitrile and 0,08% trifluoroacetic acid in water. Samples were separated on Thermo Scientific RSLC3000nano LC system, coupled to Thermo Scientific Orbitrap Exploris 480 mass spectrometer. Applied method was dependent on used Mw cutoff, relying on previous optimization efforts.

For 3 kilodaltons (kDa) eluate: Chromatography was performed by concentrating peptides on 0.3x5mm C18 PepMap trap column (Thermo Scientific) at flow of 5µl/min for 10min and further separation on 0.075mm × 250mm 2um C18 PepMap RSLC column (Thermo Scientific). Loading buffer was identical to sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 20% in 40 minutes followed with increase to 60% in 50 minutes, washing with 99% B and re-equilibration to initial conditions. Column temperature was set to 35 °C. Data were acquired in DDA (data-dependent acquisition) mode, using full scan with 120k resolution, 350-1200 Th mass range, 300% AGC (automatic gain control) target and automatic maximum injection time. Full scans were saved as profile spectra. For fragmentation minimum intensity of 5000 were selected and dynamic exclusion was set to 20s within +/-10ppm (parts per million) range from fragmented precursor and its isotopes. Charge states from 2 to 6 were allowed for fragmentation. Ion isolation window was set to 2 Th, resolution to 60k and normalized HCD (higher energy collisional dissociation) fragmentation energy to 30%. AGC (automatic gain control) target was set to Standard and scan range and maximum ion injection time to Auto.

For 10 kilodaltons (kDa) eluate: Chromatography was performed by concentrating peptides on 0.3x5mm C18 PepMap trap column (Thermo Scientific) at flow of 5µl/min for 10min and further separation on 0.075mm × 250mm 2um C18 PepMap RSLC column (Thermo Scientific). The loading buffer was identical to sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 40% in 90 minutes followed with, washing with 99% B and re-equilibration to initial conditions. Column temperature was set to 60 °C. Data were acquired in DDA (data-dependent acquisition) mode, using full scan with 120k resolution, 350-1800 Th mass range, 300% AGC (automatic gain control) target and automatic maximum injection time. Full scans were saved as profile spectra. For fragmentation minimum intensity of 3000 were selected and dynamic exclusion was set to 20s within +/-10ppm (parts per million) range from fragmented precursor and its isotopes. Fragmentation settings were dependent on ion charge. For 2 to 5 charge state ion isolation window was set to 2 Th, resolution to 30k and normalized HCD (higher energy collisional dissociation) fragmentation energy to 30%. AGC (automatic gain control) target was set to standard, and scan range and maximum ion injection time to Auto. For 6 to 10 charge state ion isolation window was set to 3 Th, resolution to 60k and normalized HCD (higher energy collisional dissociation) fragmentation energy to 30%. AGC (automatic gain control) target was set to 300%, scan range to 150-2000 Th and maximum ion injection time to Auto. 2 micro scans were collected and fragmentation scans were saved as profile spectra.

For 30 kilodaltons (kDa) eluate: Chromatography was performed by concentrating peptides on 0.3x5mm C18 PepMap trap column (Thermo Scientific) at flow of 5µl/min for 10min and further separation on 0.075mm × 250mm 2um C18 PepMap RSLC column (Thermo Scientific). The loading buffer was identical to sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 40% in 90 minutes followed with, washing with 99% B and re-equilibration to initial conditions. Column temperature was set to 50 °C.

Data were acquired in DDA (data-dependent acquisition) mode, using full scan with 120k resolution, 350-1800 Th mass range, 300% AGC (automatic gain control) target and automatic maximum injection time. Full scans were saved as profile spectra. For fragmentation minimum intensity of 3000 were selected and dynamic exclusion was set to 20s within +/-10ppm (parts per million) range from fragmented precursor and its isotopes.

Fragmentation settings were dependent on ion charge. For 2 to 5 charge state ion isolation window was set to 2 Th, resolution to 30k and normalized HCD (higher energy collisional dissociation) fragmentation energy to 30%. AGC (automatic gain control) target was set to standard, and scan range and maximum ion injection time to Auto. For 6 to 14 charge state ion isolation window was set to 3 Th, resolution to 60k and normalized HCD (higher energy collisional dissociation) fragmentation energy to 30%. AGC (automatic gain control) target was set to 300%, scan range to 150-2000 Th and maximum ion injection time to Auto. 2 micro scans were collected and fragmentation scans were saved as profile spectra.

### c) LC/MS (Liquid Chromatography with tandem mass spectrometry) for melanoma experiment, DDA (data-dependent acquisition) and DIA (data-independent acquisition)

LC (liquid chromatography) conditions were identical for DDA (data-dependent acquisition) and DIA (data-independent acquisition) experiments. Samples were separated on Thermo Scientific RSLC3000nano LC system, coupled to Thermo Scientific Orbitrap Exploris 480 mass spectrometer. Chromatography was performed by concentrating peptides on 0.3x5mm C18 PepMap trap column (Thermo Scientific) at flow of 5µl/min for 10min and further separation on 0.075mm × 250mm 2um C18 PepMap RSLC column (Thermo Scientific). Loading buffer was identical to sample dissolution buffer, mobile phase A was 2,5% acetonitrile and 0,1% formic acid in water, and mobile phase B was 80% acetonitrile in 0.1% formic acid in water. Subsequently, peptides were separated by increasing from 2,5% mobile phase B to 40% in 90 minutes followed with washing with 99% B and re-equilibration to initial conditions.

DDA (data-dependent acquisition) data were acquired using full scan with 120k resolution, 350-1800 Th mass range, 300% AGC (automatic gain control) target and automatic maximum injection time. Full scans were saved as profile spectra. For fragmentation minimum intensity of 3000 were selected and dynamic exclusion was set to 20s within +/-10ppm (parts per million) range from fragmented precursor ant its isotopes. Fragmentation settings were dependent on ion charge. For 2 to 5 charge state ion isolation window was set to 2 Th, resolution to 30k and normalized HCD (higher energy collisional dissociation) fragmentation energy to 30%. AGC (automatic gain control) target was set to Standard and scan range and maximum ion injection time to Auto. For 6 to 10 charge state ion isolation window was set to 2 Th, resolution to 60k and normalized HCD (higher energy collisional dissociation) fragmentation energy to 30%. AGC (automatic gain control) target was set to 300% and scan range and maximum ion injection time to Auto. Spectra were saved in profile mode.

DIA (data-independent acquisition) data were a acquired using full scan with 60k resolution, 350-1450 Th mass range, 300% AGC (automatic gain control) target and 100ms maximum injection time. DIA (data-independent acquisition) used 62 12 Th windows with 1 Th overlaps, covering mass range from 350 to 1200 Th. The resolution was set o 30k, AGC (automatic gain control) to 1000%, and HCD (higher energy collisional dissociation) fragmentation energy to 30%, using 3+ as the default ion charge state. Spectra were saved in profile mode.

### Data analysis procedure

Peptide/protein identification pipeline was dependent on the sub-experiments, and for clarity, they are described separately below.

### a) For comparison of different denaturation methods

Raw data files were converted to .mzml files using MSConvert v3.0.20236 with centroiding. These files were searched against the complete human Uniprot database (SwissProt+Trembl, all isoforms), concatenated with decoy reverse sequences and contaminants, using MSFragger v3.7 search engine integrated into FragPipe v18.0. Search settings were as follows. Precursor ion mass tolerance was set to 10 ppm (parts per million), and fragment mass tolerance was automatically determined. Allowed nonspecific peptide length was set to 7 to 60AA. Methionine oxidation and peptide N-term acetylation were set as flexible modifications. Automatic mass calibration and parameter optimization was allowed. Maximum allowed fragment charge was set to 3+. Search engine results were filtered and validated using Philospher, PeptideProphet and ProteinProphet built-in Fragpipe. After search, all figures were prepared using peptide spectrum matches (PSM) data extracted from psm.tsv output file. Physiochemical properties of the peptides were calculated using Python package Bio (SeqUtils.ProtParam).

### b) For comparison of different ultrafilter sizes and peptide fractionation experiment

For bottom-up search, raw data files were converted to .mzml files using MSConvert v3.0.20236 with centroiding. For visualization of peptide mass range, these files were subsequently deconvoluted using FLASHDeconv tool from OpenMS package v2.6.0. These files were searched against complete human Uniprot database (SwissProt+Trembl, all isoforms), concatenated with decoy reverse sequences and contaminants, using MSFragger v3.7 search engine integrated into FragPipe v18.0. Two searches were performed. For first search precursor ion mass tolerance was set to 8 ppm (parts per million), and fragment mass tolerance was automatically determined. Allowed nonspecific peptide length was set to 7 to 40AA. Methionine oxidation and peptide N-term acetylation were set as flexible modifications. Automatic mass calibration and parameter optimization were allowed. Maximum allowed fragment charge was set to 3+. Search engine results were filtered and validated using Philospher, PeptideProphet and ProteinProphet built-in Fragpipe. For second search precursor ion mass tolerance was set to 8 ppm (parts per million), and fragment mass tolerance was set to 10ppm (parts per million). Allowed nonspecific peptide length was set to 30 to 80AA. Methionine oxidation and peptide N-term acetylation were set as flexible modifications. Automatic mass calibration and parameter optimization were disabled. Maximum allowed fragment charge was set to 3+. Search engine results were filtered and validated using Philospher, PeptideProphet and ProteinProphet built-in Fragpipe. These searches are referred as `Normal' and 'Long' in subsequent method descriptions.

The additional top-down search was performed using InformedProteomics Package. Firstly, .pbf files were created directly from raw files using PbfGen. Subsequently, ProMex was used for data deconvolution and feature extraction, allowing for charge states 2+ to 30+ and binning resultion of 8 ppm (parts per million). The resulting files were searched against Uniprot human database containing only Swissprot sequences and their isoforms. Search parameters were as follows. Sequence length was set to 30 to 300AA, allowed charge states from 2+ to 30+ and allowed mass range between 3000 and 30 000 Da, and single internal cleavage was allowed. Maximum fragment charge was set to 15. Precursor and fragment tolerances were set to 8ppm (parts per million). Methionine oxidation and peptide N-term acetylation were set as flexible modifications, with maximum of 4 modification per peptide. Obtained spectra identifications were concatenated, and all nonconflicting (peptide spectrum matches) PSMs were retained. This may increase overall identification false discovery rate (FDR) to around 0.05 (an FDR cutoff of 0.01 was used for all searches, relying on respective analysis package predictions).
Each cellular sample were run in one DDA (data-dependent acquisition) and two DIA (data-independent acquisition) technical replicates to ensure the highest LC-MS/MS (Liquid Chromatography with tandem mass spectrometry) assay quality. DDA (data-dependent acquisition) data were used to generate the spectral library that is not only used for qualitative peptidomics but also later necessary to extract quantitative data from DIA (data-independent acquisition) files.

DDA (data-dependent acquisition) methods provide high peptide discovery potential but suffer from the lack of identification replicability and low quantification accuracy, as the rely purely on MS1 signal intensities. Identification of full sequence of particular polypeptide is especially important for developing of possible applications of particular molecules. Another aspect of the invention provides the comprehensive identification of intracellular peptides from the melanoma cellular model. The number of peptides identified in each group (WT and KO) of biological conditions have high overlap (figure 3a A, B). For further analysis, the invention considered only peptides fully quantified in the wild type (WT) and TP53 knockout (KO) samples. It has shown (figure 3a) the identification with excellent reproducibility. Venn diagram (figure3a C)comparing peptide hits between two groups (wild type/WT vs TP53 knockout/KO). The DDA (data-dependent acquisition) analysis showed (figure 3a C) identified a total of 18183 peptides with high confidence of statistical filtrations (false discovery rate/FDR 1%, 6 samples, wild type (WT) and TP53 knockout/KO). As shown in figure 3a C, a total of -11500, and -12900 cellular peptide sequences were identified in wild type (WT), and TP53 knockout (KO) A375 melanoma cellular samples, respectively, with -9300 (80%) stripped peptides commonly identified. The potential of peptidomics novel approach to employ on a large set of cellular models.

The DIA (data-independent acquisition) methodology couples the reproducibility comparable with targeted methods with the compatibility with the discovery experiments. This method relies on repetitive fragmentation of defined mass ranges generating chromatograms of most of possible parent and fragment ions. DIA (data-independent acquisition) data exhibits a higher dynamic range of quantitation, which is required for samples with high level of interfering compounds. Successful application of DIA (data-independent acquisition) methods requires an instrument of high speed, sensitivity and mass accuracy. Acquisition speed must match the narrow chromatography peak width to provide sufficient data points per peak. A faster and more sensitive mass spectrometer reduces fragmentation window size and/or widens the mass range. High sample quality is a prerequisite for any successful analysis, especially if quantification is necessary.

In the invention, sensitivity is of the utmost importance as many interesting, potentially biomarker peptides are present in trace amounts. Mass accuracy up to several ppm (parts per million) on MS and MS/MS levels is desirable, especially for DDA (data-dependent acquisition) library generation, as it is virtually necessary for efficient identification of nonspecific peptides and enables *de novo* peptide sequencing. Inventors have applied it on Orbitrap Exploris 480 instrument, which is exceptionally well-suited for this type of analyses. Inventors have decided to use the DIA (data-independent acquisition) approach for intracellular peptidomics quantification with DDA-based spectral library generation to maximize the discovery potential of whole workflow. This enables efficient and flexible peptide identification by a combination of results from multiple peptide search engines, both database-dependent and based on *de novo* sequencing approaches.

### Example 4

The invention highlights other crucial features of intracellular peptidomics analysis. In the invention DDA (data-dependent acquisition) screen, such as peptide length distribution (figure 4). The peptide length distribution depends on interest; it can easily tune and enrich the short and longer peptides by using 3, 10, 30 kilodaltons (kDa) ultrafiltration cutoff membranes. Hence invention provides the methodology that allows for the identification of peptides in a wide range of sizes. The summary of peptide-detected intensities with 3, 10, 30 kilodaltons (kDa) ultrafiltration cutoff membranes are demonstrated in figures 5A, B.

### Example 5

In this invention, in addition to peptide length distributions, inventors have evaluated the range of charge variants in the intracellular peptidomics analysis. Intracellular peptides were identified by Fragpipe, searching against the human Uniprot database with a 7-60AA length range while allowing methionine oxidation and peptide N-term acetylation as variable modifications. Interestingly, figure 6 shows that intracellular peptides exhibit a wide range of ionization states, including 2-7 charge peptides, with the majority of peptides identified with between 2-4 charge states. Identification efficiency of peptides with charge >4 may be further expanded using additional search methods. These findings could have potential applications in discovering longer peptides, intact proteins, or even small-size proteins. However, it is necessary to use a specific methodology to work with very complex spectra of long peptides and small proteins.

### Example 6

The peptidome data generated with the invention approach shows high consistency and reproducibility between biological replicates. Peptides were quantified by DIA-NN using a spectral library generated by FragPipe. Results show that most peptides can be quantified without missing values (figure 7). It means most of the peptides quantified with valid value six, which shows highly confident quantification qualities.

### Example 7

Above examples highlighted that the high number of valid values on the precursor level means higher confidence in peptidome quantitative analysis. The next and crucial point is to demonstrate the peptide-level quantification reproducibility. Therefore, the intracellular peptides were quantified by DIA-NN using a spectral library generated by Fragpipe. Particularly here, only fully quantified precursors were selected and averaged over charges state and technical replicate. This led to the quantification of 16076 peptides. Scatterplots of biological replicate measurements with Pearson R² correlation coefficient are higher than 0.8 and 0.9 (0.8 and 0.9 > R² ) which show high level of correlation for peptide level quantification, a good level of reproducibility between samples (biological replicated of WT and KO cellular samples) (figure 8). Figure 8 reveals that inventors were able to apply DIA (data-independent acquisition) quantification for intracellular peptidome samples obtained with the invention of simple peptide extraction, purification, and data analysis workflow. The quantification results were highly reproducible, so it was further possible to detect differentially abundant peptides with reasonable sensitivity.

### Example 8

The further analysis is wholly based on intracellular peptide quantitation. In a quantitative experiment, first used DDA (data-dependent acquisition) data to generate an appropriate spectral library not losing many important cellular peptides while not introducing too many low-confidence cellular peptides. Then, exclusively DIA (data-independent acquisition) data was used to extract quantitative information about peptides included in the spectral library. Furthermore, consider DIA (data-independent acquisition) data as an only choice to properly quantitate cellular peptides due to its perfect reproducibility multiplexing. Moreover, as already suggested, DIA (data-independent acquisition) data could be used for both the identification and precise quantitation of cellular peptides. Several methods with distinct window widths were developed for accurate quantitation of TP53-dependent markers in the melanoma model. The peptide precursor ions distribution was initially screened in the optimized DDA (data-dependent acquisition) method from cellular peptidome samples. The inventors determined the most populated part of precursor range covering most of the observed peptide masses. The rest of the DIA (data-independent acquisition) method parameters such as normalized AGC (Automatic Gain Control) target, injection time, window overlap, were optimized to keep the cycle time up to 4.5 s. Such method cycle in combination with described chromatographic conditions (in method section) will yield at least 10 data points per peak as is a minimum recommendation by FDA (The United States Food and Drug Administration) at the same time keeping sufficient selectivity and sensitivity of the method.

### Peptide quantification - data analysis for quantitative peptidomics of melanoma A375 cells

Data were analyzed mostly with the goal of quantitative characterization of intracellular peptidome. Raw data files (DDA (data-dependent acquisition) and DIA (data-independent acquisition) runs) were converted to .mzml files using MSConvert v. with centroiding. Converted MS data were searched using MSFragger 3.4 embedded in Fragpipe suite against complete Homo sapiens Uniprot database (SwissProt+Trembl, all isoforms) concatenated with indexed retention time (iRT) peptide sequences, decoy reverse sequences and contaminants. The search database was constructed in Fragpipe v. suite. Additional DDA-like data was extracted from DIA (data independent acquisition) files by DIA-Umpire signal extraction engine integrated in Fragpipe. The following search settings were used for MSFragger: precursor mass tolerance was set to +/-10 parts per million (ppm) and fragment mass tolerance was automatically optimized. Enzyme digestion was set to nonspecific and peptide length was set from 7 to 60 amino acids. Peptide mass range was set from 500 to 8000 Dalton. Variable modifications were set to: methionine oxidation, peptide N-term acetylation. Maximum allowed fragment charge was set to3. Data were mass recalibrated, and automatic parameter optimization setting was used to tune fragment mass tolerance. Search result filtration and validation were done automatically by Philosopher. EasyPQP were used to create the spectral library for further use in DIA-based quantification. Two obtained spectra libraries were aligned by retention time using custom Python script. DIA (data-independent acquisition) data processing were performed using DIA-NN v 1.8.1, All .mzml files were converted to .dia files. MBR option was selected. Precursor false discovery rate (FDR) was set to 1 %. Robust LC (high accuracy) option was selected. MS2 and MS1 mass accuracies, and scan window was set to 14 ppm (parts per million), 11 and 7, respectively - values were chosen as suggested by DIA-NN after analyzing each run separately. Retention time (RT)-dependent cross-run normalization was applied to data. Other settings in the tab were left default. Peptide quantification data were prepared by diann-r R library. Data were preprocessed in Python to average technical replicates.
Results of DIANN quantification of peptidomes extracted from wild-type (WT) and TP53 knockout (KO) A375 melanoma cells.

Quantification data obtained by DIA-NN were post-processed in Perseus. Significantly regulated peptides were selected by using t-test and permutation-based FDR (false discovery rate) correction at 1% FDR. Results were visualized as volcano plots provided in figure 9. In the melanoma cellular model, gene TP53-dependent up and downregulated peptides and their source of the proteins are listed in the table 2a, b, respectively. Volcano plots show that this peptide quantitation experiment provides a list of peptides that significantly stratify between compared melanoma cellular groups. The following tables summarize peptides that have shown significant regulation in the analysis described above.

**Table 2: The list of intracellular peptides, 2a) upregulated and 2b) downregulated in TP53 knockout (KO) A375 melanoma cells.**

| log2 WT-KO | Genes | First Protein Description | Modified Sequence |
|---|---|---|---|
| -2.27108 | RPS27 | 40S ribosomal protein | PLAKDLLHPSPEEEKRKHKKKRL |
| | | S27 | VQ |
| -1.66888 | HSPB 1 | Heat shock protein beta-1 | APAYSRAL |
| -1.71294 | HSPB 1 | Heat shock protein beta-1 | RAQLGGPEAAKSDETAAK |
| -1.57424 | HSPB 1 | Heat shock protein beta-1 | SPAVAAPAYSRALSRQL |
| -2.20141 | PCNA | Proliferating cell nuclear antigen | TSMSKILKC |

| Table 2a. The list of peptides upregulated in TP53 knockout (KO) cells | | | |
|---|---|---|---|
| log2 WT-KO | Genes | First Protein Description | Modified Sequence |
| 2.40412 | YWHAZ | 14-3-3 protein zeta/delta | ISKKEMQPTHPIR |
| 4.15191 | RPS11 | 40S ribosomal protein S11 | (UniMod: 1)ADIQTERAYQKQPT |
| 3.61133 | RPLP1 | 60S acidic ribosomal protein P1 | GPAPAAGAAPAGGPAPSTAAAPA EEKK |
| 4.66107 | RPLP2 | 60S acidic ribosomal protein P2 | AAPGSAAPAAGSAPAAAEEKK |
| 3.80898 | RPLP2 | 60S acidic ribosomal protein P2 | AAPGSAAPAAGSAPAAAEEKKDE KK |
| 3.44036 | RPLP2 | 60S acidic ribosomal protein P2 | AAPGSAAPAAGSAPAAAEEKKDE KKE |
| 2.3719 | RPL29 | 60S ribosomal protein L29 | APASVPAQAPKRTQAPT |
| 2.79006 | ACTC1 | Actin, alpha cardiac muscle 1 | YVGDEAQSKRGILTLKY |
| 2.28008 | ACTG1 | Actin, cytoplasmic 2 | KQEYDESGPSIVHRK |
| 3.48896 | CAVIN3 | Caveolae-associated protein 3 | EPEPPQDTEEDPGRPG |
| 4.21344 | EEF1A1 | Elongation factor 1-alpha 1 | DVRRGNVAGDSKNDPP |
| 3.22707 | EEF1A1 | Elongation factor 1-alpha 1 | LPPTRPTDKPLR |
| 3.3696 | EEF1A1 | Elongation factor 1-alpha 1 | RVETGVLKPG |
| 2.71513 | EEF1G | Elongation factor 1-gamma | AEPKAKDPFAH |
| 2.45305 | HSPA5 | Endoplasmic reticulum chaperone BiP | DAAKNQLTSNPENT |
| 2.38004 | HSPA5 | Endoplasmic reticulum chaperone BiP | EPSRGINPDEA |
| 2.91579 | HSPA4 | Heat shock 70 kDa protein 4 | GPVDGQGDNPGPQAAEQGTDTA VPSDSDKK |
| 3.98777 | HSP90A B1 | Heat shock protein HSP 90-beta | KEGLELPEDEEEKK |
| 3.65985 | HSP90A B1 | Heat shock protein HSP 90-beta | KEGLELPEDEEEKKK |
| 3.28967 | CANX | Isoform 2 of Calnexin | RPVIDNPNYK |
| 2.12478 | GSTK1 | Isoform 2 of Glutathione S-transferase kappa 1 | GPLPRTVELF |
| 3.55759 | GLG1 | Isoform 2 of Golgi apparatus protein 1 | LPGQGVHSQGQGPG |
| 3.33983 | HSP90A A1 | Isoform 2 of Heat shock protein HSP 90-alpha | KEGLELPEDEEEKKKQEEKK |
| 4.50283 | PAGES | Isoform 2 of P antigen family member 5 | (UniMod:1)SEHVTRSQSSERGNDQ ESSQPVGP |
| 3.56553 | RANBP1 | Isoform 2 of Ran-specific GTPase-activating protein | (UniMod:1)AAAKDTHEDHDTSTEN TDESNHDP |
| 4.23428 | RANBP1 | Isoform 2 of Ran-specific GTPase-activating protein | (UniMod:1)AAAKDTHEDHDTSTEN TDESNHDPQ |
| 4.59966 | UBQLN1 | Isoform 2 of Ubiquilin-1 | ATPSENTSPTAGTTEPGHQQ |
| 4.60047 | UBQLN1 | Isoform 2 of Ubiquilin-1 | TPSENTSPTAGTTEPGHQQ |
| 3.52008 | NCOA4 | Isoform 3 of Nuclear receptor coactivator 4 | KEGKDKNGMPVEPKPEPEKHK |
| 3.10353 | RTN4 | Isoform B of Reticulon-4 | LPEDDEPPARPP |
| 3.94605 | RTN4 | Isoform B of Reticulon-4 | SPSKLPEDDEPPARPPPPPP |
| 2.47895 | RTN4 | Isoform B of Reticulon-4 | TPPAPAPAAPPSTPAAPK |
| 3.38298 | RTN4 | Isoform B of Reticulon-4 | TPPAPAPAAPPSTPAAPKR |
| 3.73938 | RTN4 | Isoform B of Reticulon-4 | VPPAPRGPLPAAPPVAPERQPS |
| 2.72782 | RTN4 | Isoform B of Reticulon-4 | VSPSKLPEDDEPPARPPP |
| 4.18262 | CLTA | Isoform Non-brain of Clathrin light chain A | APGPQPHGEPPGGPD |
| 4.81605 | LDHB | L-lactate dehydrogenase B chain | APVAEEEATVPNNK |
| 2.60434 | LDHB | L-lactate dehydrogenase B chain | NPEM(UniMod:35)GTDNDSENWK |
| 1.96861 | MVP | Major vault protein | AQDPFPLYPGEVL |
| 3.00583 | PAICS | Multifunctional protein ADE2 | (UniMod:1)ATAEVLNIGKK |
| 2.34989 | PRDX1 | Peroxiredoxin-1 | (UniMod:1)SSGNAKIGHPAP |
| 3.16052 | PPP1R14 B | Protein phosphatase 1 regulatory subunit 14B | SPPGAAGEGPGGADDEGPVR |
| 2.29595 | S100A4 | Protein S100-A4 | FEGFPDKQPRKK |
| 1.46935 | S100A4 | Protein S100-A4 | SCIAMMCNEFFEGFPDKQPRKK |
| 2.0579 | PKM | Pyruvate kinase PKM | ALDTKGPEIR |
| 4.07381 | SQSTM1 | Sequestosome-1 | ARPGPTAESASGPSEDPS |
| 3.53094 | SQSTM1 | Sequestosome-1 | ARPGPTAESASGPSEDPSVN |
| 3.4108 | SQSTM1 | Sequestosome-1 | FPSPFGH |
| 3.68989 | SQSTM1 | Sequestosome-1 | GPPGNWSPRPP |
| 5.10528 | SQSTM1 | Sequestosome-1 | IDVEHGGKRSRLTPVSPESSSTEEK SSSQPS |
| 3.88978 | SQSTM1 | Sequestosome-1 | LPPEADPR |
| 3.67784 | SQSTM1 | Sequestosome-1 | LTPVSPESSSTEEK |
| 3.949 | SQSTM1 | Sequestosome-1 | LTPVSPESSSTEEKS |
| 4.29065 | SQSTM1 | Sequestosome-1 | LTPVSPESSSTEEKSSSQPS |
| 3.29785 | SQSTM1 | Sequestosome-1 | LTPVSPESSSTEEKSSSQPSS |
| 3.48737 | SQSTM1 | Sequestosome-1 | RPGPTAESASGPSE |
| 3.93524 | SQSTM1 | Sequestosome-1 | RPGPTAESASGPSEDPS |
| 3.10187 | SQSTM1 | Sequestosome-1 | RPGPTAESASGPSEDPSVN |
| 5.03332 | SQSTM1 | Sequestosome-1 | RSRLTPVSPESSSTEEKSSSQPS |
| 3.21954 | SQSTM1 | Sequestosome-1 | SDPSKPGGNVEGATQ |
| 2.56424 | SQSTM1 | Sequestosome-1 | SDPSKPGGNVEGATQSLAEQMRK |
| 4.35323 | SQSTM1 | Sequestosome-1 | SLDPSQEGPT |
| 3.81511 | SQSTM1 | Sequestosome-1 | SPRPPRAGEARPGP |
| 3.37548 | SQSTM1 | Sequestosome-1 | SPRPPRAGEARPGPT |
| 3.67689 | SQSTM1 | Sequestosome-1 | TPVSPESSSTEEKSSSQPS |
| 2.06089 | SH3BGR L3 | SH3 domain-binding glutamic acid-rich-like protein 3 | AVEQNTLQEFLKLA |
| 1.64742 | SH3BGR L3 | SH3 domain-binding glutamic acid-rich-like protein 3 | VEAVEQNTLQEFLKLA |
| 3.02239 | VAT 1 | Synaptic vesicle membrane protein VAT-1 homolog | SRPAAPPAPGPG |
| 2.42432 | YBX1 | Y-box-binding protein 1 | RQPREDGNEEDKENQGDETQGQQ PPQR |
| Table 2b. The list of peptides downregulated in TP53 knockout (KO) cells | | | |

### Example 9

To further understand biological significance inferred from cellular peptidome quantitative data in wild type (WT) and TP53 knockout (KO) A375 melanoma cells, inventors have employed bioinformatics approaches such as Search Tool for the Retrieval of Interacting Genes/Proteins (STRING). Initially, inventors generated interactome maps using the STRING server to determine characteristic nodes enriched in TP53 knockout cells. It aims to analyze significantly dysregulated protein identifiers in TP53 knockout cells. Source genes of significantly regulated peptides and TP53 were used as input to STRING network visualization and analysis server (It showed a very close relation between selected protein identifiers with the enrichment of protein-protein interaction p-value less than 1.0e-16. Analysis shows an interesting Search Tool for Retrieving Interacting Genes/Proteins (STRING) nodes characteristic for TP53 knockout A375 melanoma cells (figure 10).
16076 quantified peptides were mapped to 1579 protein precursors. As shown in figure 10, analysis reveals the main terms related to TP53-dependent protein regulation in the melanoma cellular model. Thus, STRING interaction networks provide another potent way of determining protein nodes from which a predictive role of the intracellular peptide could be determined.

The intracellular peptidomics approach allows us to screen the peptide level expression in different cellular conditions. The screening facilitates understanding the exact biological mechanism and the discovery of peptide biomarkers for various disease conditions. Therefore, the final analysis of intracellular peptidomics shows that differential abundance profile of multiple peptides between the groups. Invention quantitative data shows even single proteins is the source of various intracellular peptides that may have differential abundance profiles. Inventors are able to quantify these fascinating examples, namely Sequestosome-1 (SQSTM1), Ran-specific GTPase-activating protein (RANG) and Heat shock protein beta-1 (HSPB1) are sources of subsets of differentially abundant peptides detected in melanoma A375 TP53 knockout (KO) compared with wild type (WT) experiment (figure 11a).
In addition, the intracellular peptidomics approach clearly discriminates the peptides which are differentially regulated and non-regulated peptides even though the source of the protein is the same (figure 11b). It suggests that there are differential isoform degradation, partial degradation or other more complex process concerning these proteins in this cellular model. Therefore, these correlations between different peptides with potentially identical sources can be helpful for the biological interpretation of the results.

### Example 10

### Hydrophilic-lipophilic balanced columns fractionation of intracellular peptides

The first steps are similar to typical peptide isolation described in Example 1. For fractionation, column with loaded sample was washed with 3x 1ml of 5% MeOH in 0,2% formic acid, and subsequently eluted in six fractions using 10%, 20%, 40%, 60%, 80% of methanol in 0.2% formic acid volume per volume (v/v) (in this solution final proportion of methanol and formic acid in water was 10%, 20%, 40%, 60%, 80% and 0.2%, respectively) and 90% acetonitrile in 1% formic acid volume per volume (v/v) (in this solution the final proportion of acetonitrile and formic acid in water was 90% and 1%, respectively). Eluates were dried under vacuum, resuspended in 2.5 % acetonitrile, 0.2% TFA and analyzed by LC/MS (Liquid Chromatography with tandem mass spectrometry) using 30 min gradients. Total analysis time was less than 12 hours, including blank runs. The wider length distribution (7 to 130 amino acids long) of peptides identified in all fractions shown in figure 12B.
To identify longer peptides, inventors applied an additional analysis tool, the Informed Proteomics suite, and concatenated results with Fragpipe search results.
Fractionation efficiency is shown in figure 12. The total number of peptides identified in this analysis was 4041. As analysis of the most abundant fraction yielded 1559 peptide identifications, inventors clearly state that fractionation with methanol steps was efficient and useful for the identification of peptides in a wide mass range. Notably, only 5-10 million cells were used for fractionation on a single 30mg hydrophilic-lipophilic balanced columns column, and signal intensity observed during analysis indicated practically maximal column loading. Therefore, using this method, it is not necessary to use an extensive amounts of starting material. This application shows a high potential for customization and development of the proposed methodology.

In conclusion, the invention includes the following key highlights;
1. Method of sample preparation for qualitative and quantitative analysis of intracellular peptidome in mammalian cells. It is based on amino acid sequencing with tandem mass spectrometry with signaling intensities measurement of peptides characterized in that the method comprises only two steps of sample preparation, a) urea denaturation of mammalian cells, preferably continuous cellular models, primary cells isolated from tissue, and blood samples where a solution of urea (purity ≥99.5%) in phosphate-buffered saline (PBS) without additives (no calcium/magnesium/phenol red) pH 7.2-7.5 is used 20-30 minutes for sonication (ultrasonic bath sonicator without heat) and vortexing at room temperature (RT) to lyse the cells and dissociate protein-peptide interactions;
   b) purification by oasis columns (hydrophilic-lipophilic balanced) for 120-180 minutes at room temperature (RT) to eliminate the abundant proteins and simultaneously elute the cellular peptides further separated with an ultrafiltration approach using selected molecular weight cutoff filter (3 or 10 or 30 kilo daltons, kDa) for 15-30 minutes, 4 - 10 °C and removal of high- molecular weight proteins and other cellular compounds (> 30 kDa). Then peptides are subjected to the liquid chromatography coupled with mass spectrometry (LC/MS) measurement and qualitative, quantitative data analysis.
2. Method of urea (purity ≥99.5%) of 8 M (molar) dissolved in phosphate-buffered saline (PBS) without additives (no calcium/magnesium/phenol red) pH 7.2-7.5 tested and selected for denaturation of intracellular peptides, which has shown superior performance than 200 µl of 50% ACN (acetonitrile) and 50 µl of 100% FA (formic acid) agents.
3. The intracellular peptides are obtained in various sizes according to the molecular weight filters (3 or 10 or 30 kilo daltons, kDa) used to collect intracellular peptides.
4. The invention method can be applied to all mammalian cells.
5. In the invention, additional 2-6 elutions/fractionations can be used to enrich of longer (7 - 130 amino acids) size of intracellular peptides.

### References

[1] R. Richter et al., "Composition of the peptide fraction in human blood plasma: database of circulating human peptides," J. Chromatogr. B. Biomed. Sci. App., vol. 726, no. 1, pp. 25-35, Apr. 1999, doi: 10.1016/S0378-4347(99)00012-2.
[2] T. Cardon et al., "Optimized Sample Preparation Workflow for Improved Identification of Ghost Proteins," Anal. Chem., 2020, doi: 10.1021/acs.analchem.9b04188.
[3] B. Fabre, J.-P. Combier, and S. Plaza, "Recent advances in mass spectrometry-based peptidomics workflows to identify short-open-reading-frame-encoded peptides and explore their functions," Curr. Opin. Chem. Biol., vol. 60, pp. 122-130, Feb. 2021, doi: 10.1016/j.cbpa.2020.12.002.
[4] J. Rimbon, A. Sánchez-Kopper, A. Wahl, and R. Takors, "Monitoring intracellular protein degradation in antibody-producing Chinese hamster ovary cells," Eng. Life Sci., vol. 15, no. 5, pp. 499-508, Jul. 2015, doi: 10.1002/elsc.201400103.
[5] X. Li et al., "Comparative peptidome profiling reveals critical roles for peptides in the pathology of pancreatic cancer," Int. J. Biochem. Cell Biol., vol. 120, p. 105687, Mar. 2020, doi: 10.1016/j.biocel.2020.105687.
[6] S. Dasgupta et al., "Proteasome Inhibitors Alter Levels of Intracellular Peptides in HEK293T and SH-SY5Y Cells," PLOS ONE, vol. 9, no. 7, p. e103604, Jul. 2014, doi: 10.1371/journal.pone.0103604.
[7] C.-X. Zhou, M. Gao, B. Han, H. Cong, X.-Q. Zhu, and H.-Y. Zhou, "Quantitative Peptidomics of Mouse Brain After Infection With Cyst-Forming Toxoplasma gondii," Front. Immunol., vol. 12, 2021, Accessed: Feb. 15, 2023. [Online]. Available: https://www.frontiersin.org/articles/10.3389/fimmu.2021.681242
[8] B. S. Parmar et al., "Identification of Non-Canonical Translation Products in C. elegans Using Tandem Mass Spectrometry," Front. Genet., vol. 12, 2021, Accessed: Feb. 15, 2023. [Online]. Available: https://www.frontiersin.org/articles/10.3389/fgene.2021.728900
[9] L. Zhang et al., "Peptidomics Analysis Reveals Peptide PDCryab1 Inhibits Doxorubicin-Induced Cardiotoxicity," Oxid. Med. Cell. Longev., vol. 2020, p. 7182428, Oct. 2020, doi: 10.1155/2020/7182428.
[10] L. Wu et al., "Peptidomic Analysis of Cultured Cardiomyocytes Exposed to Acute Ischemic-Hypoxia," Cell. Physiol. Biochem., vol. 41, no. 1, pp. 358-368, 2017, doi: 10.1159/000456282.
[11] Y. Xue et al., "Peptidomic Analysis of Endometrial Tissue from Patients with Ovarian Endometriosis," Cell. Physiol. Biochem., vol. 47, no. 1, pp. 107-118, 2018, doi: 10.1159/000489753.
[12] G. P. Roberts et al., "Comparison of Human and Murine Enteroendocrine Cells by Transcriptomic and Peptidomic Profiling," Diabetes, vol. 68, no. 5, pp. 1062-1072, May 2019, doi: 10.2337/db18-0883.
[13] Y. Li et al., "Identification of intracellular peptides associated with thermogenesis in human brown adipocytes," J. Cell. Physiol., vol. 234, no. 5, pp. 7104-7114, 2019, doi: 10.1002/jcp.27465.
[14] Y. Gao et al., "Identification and characterization of metformin on peptidomic profiling in human visceral adipocytes," J. Cell. Biochem., vol. 119, no. 2, pp. 1866-1878, 2018, doi: 10.1002/jcb.26347.
[15] P. Broberg, T. Fehniger, C. Lindberg, and G. MARKO-VARGA, "Peptides as biomarkers of copd," WO2006118522A1, Nov. 09, 2006 Accessed: Feb. 15, 2023. [Online]. Available: https://patents.google.com/patent/WO2006118522A1/en?oq=WO2006%2f118522A 1
[16] J. Klein, M. L. Merchant, R. Ouseph, and R. A. Ward, "Peptide biomarkers of cardiovascular disease," US20140243432A1, Aug. 28, 2014 Accessed: Feb. 15, 2023. [Online]. Available: https://patents.google.com/patent/US20140243432A1/en?oq=US2014%2f0243432A 1
[17] C. Watson, M. Ledwidge, K. McDonald, and J. Baugh, "Biomarkers of cardiovascular disease including Irg," US20130084276A1, Apr. 04, 2013 Accessed: Feb. 15, 2023. [Online]. Available: https://patents.google.com/patent/US20130084276A1/en?oq=US2013%2f0084276A 1
[18] T. GARCÍA-BERROCOSO and J. M. Vilallonga, "Biomarkers for the prognosis of ischemic stroke," WO2014064202A1, May 01, 2014 Accessed: Feb. 15, 2023. [Online]. Available: https://patents.google.com/patent/WO2014064202A1/en?oq=WO2014%2f064202A 1

## Claims

1. Method of sample preparation for qualitative and quantitative analysis of intracellular peptidome in mammalian cells that is based on amino acid sequencing with tandem mass spectrometry with signaling intensities measurement of peptides **characterized in that** the method comprises two steps of sample preparation as follows:
a) urea denaturation of the sample of mammalian cells, preferably continuous cellular models, primary cells isolated from tissue, and blood samples, where a solution of urea at a purity ≥99.5% in phosphate-buffered saline (PBS) pH 7.2-7.5 is used for 20-30 minutes for sonication and mixing, preferably by vortexing at room temperature to lyse the cells and dissociate protein-peptide interactions;
b) purification by oasis columns of hydrophilic-lipophilic balanced for 120-180 minutes at room temperature to eliminate the abundant proteins and simultaneously elute the cellular peptides further separated with an ultrafiltration using selected molecular weight cutoff filter that has 3-30 kilo daltons kDa, preferably 3 or 10 or 30, kDa for 15-30 minutes at 4 - 10 °C and removal of high- molecular weight proteins and other cellular compounds that have > 30 kDa;

2. Method of claim 1, wherein the method comprises the following steps:
a) urea denaturation of mammalian cells, where a solution of urea (purity ≥99.5%) in phosphate-buffered saline (PBS) without additives (no calcium/magnesium/phenol red) pH 7.2-7.5 is used 20-30 minutes for sonication (ultrasonic bath sonicator without heat) and vortexing at room temperature (RT) to lyse the cells and dissociate protein-peptide interactions;
b) purification by oasis columns (hydrophilic-lipophilic balanced) for 120-180 minutes at room temperature (RT) to eliminate the abundant proteins and simultaneously elute the cellular peptides further separated with an ultrafiltration approach using selected molecular weight cutoff filter of 3 or 10 or 30 kilo daltons, kDa for 15-30 minutes, 4 - 10 °C and removal of high- molecular weight proteins and other cellular compounds of > 30 kDa). Then peptides are subjected to the liquid chromatography coupled with mass spectrometry (LC/MS) measurement and qualitative, quantitative data analysis.

3. Method of claim 1, wherein denaturation step is performed with only urea of ≥99.5% purity and 8 M concentration in phosphate-buffered saline (PBS) of pH 7.2-7.5 without additives as calcium/magnesium/phenol red.
